# EUROPEAN PATENT SPECIFICATION

(11) **EP 4 115 862 B1**
(45) Date of publication and mention of the grant of the patent: **22.10.2025**
(21) Application number: 21796407.1
(22) Date of filing: 26.04.2021
(51) Int. Cl.: A61F 13/42, A61F 5/44, G01N 27/00, G01N 27/06, G01N 27/22

(54) **ABSORBENT ARTICLE AND DEFECATION AND URINATION DETERMINATION METHOD**
SAUGFÄHIGER ARTIKEL UND VERFAHREN ZUR DEFÄKATIONS- UND URINATIONSBESTIMMUNG
ARTICLE ABSORBANT ET PROCÉDÉ DE DÉTERMINATION DE DÉFÉCATION ET DE MICTION

(30) Priority: 30.04.2020 JP 2020080498
(43) Date of publication of application: 11.01.2023
(73) Proprietor: Unicharm Corporation, Ehime 799-0111 (JP)
(72) Inventor: TODA, Haruki, Kanonji-shi, Kagawa 769-1602 (JP); MURAI, Takamasa, Kanonji-shi, Kagawa 769-1602 (JP); OKUNO, Shingo, Kanonji-shi, Kagawa 769-1602 (JP)
(74) Representative: Dolleymores
(86) International application number: PCT/JP2021/016642
(87) International publication number: WO 2021/221014

(56) References cited:
- WO-A1-2015/102084
- WO-A1-2019/090387
- CN-U- 209 661 980
- JP-A- 2002 515 975
- JP-A- 2009 505 781
- JP-A- 2015 128 477
- JP-A- 2015 229 003
- JP-A- 2019 141 190

## Description

### FIELD

The present invention relates to an absorbent article and a defecation/urination determination method.

### BACKGROUND

As for conventional disposable diapers and the like, there have been known absorbent articles having an excretion detecting function for detecting excretion of urine and notifying a user thereof. For example, Patent Literature 1 discloses a technique of arranging a moisture sensor including a pair of electrodes inside an absorbent body of a diaper, making it possible to detect urination based on conducting between the electrodes by the moisture when urine is excreted.

### [CITATION LIST]

### [PATENT LITERATURE]

Patent Literature 1: Japanese Patent Application Publication No. 2012-223386

Further prior art arrangements of absorbent articles having electrodes for detecting excretion are known from CN209661980U, JP2019141190A and WO2019/090387A1.

### SUMMARY

### [TECHNICAL PROBLEM]

In recent years, in a situation where a bedridden elderly person is wearing a diaper in a nursing facility, from the viewpoint of reducing the burden on the caregiver, it has been demanded that the presence/absence of excretion and whether the excrement is feces or urine can be accurately determined even in a state in which the diaper is put on. However, in an absorbent article having a conventional excretion detecting function such as that of Patent Literature 1, the presence/absence of excretion can be detected, but it cannot be distinguished whether the excrement is feces or urine. Therefore, a caregiver has a trouble of opening and checking the diaper every time of excretion.

The present invention was achieved in light of conventional problems such as that described above, and an aspect of the present invention is to provide an absorbent article that enables to accurately determine whether excrement is feces or urine.

### [SOLUTION TO PROBLEM]

A main aspect of the present invention for achieving the above-described aspect is an absorbent article as claimed in claim 1. Preferred but optional features are defined by the dependent claims.

Features of the present invention other than the above will become clear by reading the description of the present specification with reference to the accompanying drawings.

### [ADVANTAGEOUS EFFECT OF THE INVENTION]

According to the present invention, it is possible to provide an absorbent article that enables to accurately determine whether excrement is feces or urine.

### BRIEF DESCRIPTION OF DRAWINGS

FIG. 1A is a schematic plan view showing an unfolded state of a diaper 101.
FIG. 1B is a schematic cross-sectional view showing a cross section taken along a line X-X in FIG. 1A.
FIG. 2 is a schematic plan view of an absorbent pad 1.
FIG. 3 is a schematic cross-sectional view showing a cross section taken along a line A-A in FIG. 2.
FIG. 4 is a schematic plan view and a cross-sectional view illustrating an example of a configuration of a pair of skin-side electrodes 11 and 11.
FIG. 5 is a conceptual diagram of an excrement detection circuit using the absorbent pad 1.
FIGS. 6A and 6B are diagrams illustrating the principle of detecting urine when urine is excreted.
FIGS. 7A and 7B are diagrams illustrating the principle of detecting feces when feces is excreted.
FIG. 8 is a flow chart showing an example of a defecation/urination determination process using the absorbent pad 1.
FIG. 9 is a flow chart showing an example of a defecation/urination determination process using the absorbent pad 1 in a second embodiment.

### DESCRIPTION OF EMBODIMENTS

At least following matters will become clear with description of this specification and attached drawings.

An absorbent article having a longitudinal direction, a width direction, and a thickness direction that intersect each other in a stretched state, the absorbent article including: a liquid-absorbent absorbent body; a liquid-permeable sheet that is arranged on a skin side in the thickness direction with respect to the absorbent body; a liquid-impermeable sheet that is arranged on a non-skin side in the thickness direction with respect to the absorbent body; a skin-side electrode between the liquid-permeable sheet and the absorbent body; and a non-skin-side electrode between the liquid-impermeable sheet and the absorbent body.

According to the above-described absorbent article, it is possible to measure changes in the predetermined period between a skin-side capacitance value detected by the skin-side electrode and a non-skin-side capacitance value detected by the non-skin-side electrode. Since the behaviors of the changes in the skin-side capacitance value and the non-skin-side capacitance value are different between during urination and during defecation, monitoring of the behaviors of these changes makes it possible to accurately determine whether the excrement is feces or urine.

In such an absorbent article, at least one pair of the non-skin-side electrodes are provided spaced apart from each other in the width direction by a predetermined distance.

According to the above-described absorbent article, upon attaching moisture such as urine to a portion between the pair of non-skin-side electrodes that are spaced apart from each other in the width direction, it becomes conducted between the electrodes and this changes the capacitance value and the resistance value detected between the electrodes. By measuring this amount of change, it is possible to accurately determine the excretion of urine or feces.

In such an absorbent article, at least one pair of the skin-side electrodes are provided spaced apart from each other in the width direction by a distance that is different from the predetermined distance.

According to the above-described absorbent article, similarly to the non-skin-side electrode, by measuring the amount of change in the capacitance value and the resistance value, it is possible to accurately determine the excretion of urine or feces. Further, the skin-side electrode and the non-skin-side electrode are arranged at positions displaced from each other in the width direction, and this makes it easier to perform work for connecting a detection device or the like to each of the electrodes.

In such an absorbent article, a space between the pair of skin-side electrodes in the width direction is larger than a space between the pair of non-skin-side electrodes in the width direction.

According to the above-described absorbent article, the space between the skin-side electrodes, which urine easily reaches, is large, and therefore it is possible to relatively suppress an increase in capacitance and a decrease in resistance with respect to the non-skin-side electrodes. This makes it possible to suppress that the determination based on the combination of the changes on the skin surface side and the non-skin surface side cannot be used due to previous exceeding the limit value of the skin-side electrode.

In such an absorbent article, it is desirable that a space between the pair of skin-side electrodes in the width direction is 40 mm or larger.

According to the above-described absorbent article, the space between the skin-side electrodes in the width direction is not excessively narrow, and therefore this suppresses the generation of noise caused by contact between the electrodes, for example when a diaper is applied to the narrow crotch portion. This makes it possible to suppress the occurrence of erroneous detection. Further, it is possible to make it easier to appropriately adjust the upper limit and the lower limit of the capacitance value and the resistance value detected between the electrodes.

In such an absorbent article, it is desirable that a space between the pair of non-skin-side electrodes in the width direction is 40 mm or larger.

According to the above-described absorbent article, the space between the non-skin-side electrodes in the width direction is not excessively narrow, and therefore this suppresses the generation of noise during use. This makes it possible to suppress the occurrence of erroneous detection. Further, it is possible to make it easier to appropriately adjust the upper limit and the lower limit of the capacitance value and the resistance value detected between the electrodes.

In such an absorbent article, it is desirable that a presence/absence of excretion is detected based on contact of each of the skin-side electrode and the non-skin-side electrode with excrement.

According to the above-described absorbent article, the direct contact between the excrement and the electrodes makes it possible to facilitate the flow of a current between the electrodes through moisture contained in the excrement. Therefore, compared with the case where the excrement and the electrodes do not come into contact with each other, the change in the capacitance value or the resistance value detected between the electrodes becomes clear, enabling to make determination of the excrement more accurate.

In such an absorbent article, it is desirable that the skin-side electrode and the non-skin-side electrode each include: a conductive portion; and a liquid-impermeable sheet member that is arranged overlaid on the conductive portion in the thickness direction.

According to the above-described absorbent article, on the one side of the conductive portion in the thickness direction, the contact between the conductive portion and excrement is limited by the liquid-impermeable sheet member, and on the other side of the conductive portion in the thickness direction, the contact between the conductive portion and the excrement is easily made. That is, the influence of the contact between the conductive portion and the excrement can be limited to the one side in the thickness direction. This makes it possible to select whether to detect the influence of the skin side (top sheet side) of the skin-side electrode or the influence of the non-skin side (absorbent body side) of the skin-side electrode, for example.

In such an absorbent article, it is desirable that the skin-side electrode and the non-skin-side electrode both include the liquid-impermeable sheet member on an identical side in the thickness direction.

According to the above-described absorbent article, the conductive portion of each electrode is exposed on the identical side in the thickness direction. Therefore, when connecting a detection device or the like to each electrode, the connection unit can be connected to the identical side in the thickness direction, making easier work for attaching the detection device or the like.

In such an absorbent article, it is desirable that the skin-side electrode and the non-skin-side electrode both include the liquid-impermeable sheet member on the non-skin side in the thickness direction.

According to the above-described absorbent article, the conductive portion of each electrode is exposed on the skin side in the thickness direction. Therefore, this makes the excrement excreted on the skin side of the absorbent article more likely to come into contact with the conductive portion of each electrode, enabling to make easier to accurately detect the excrement.

In such an absorbent article, it is desirable that a width of the liquid-impermeable sheet member is larger than widths of the skin-side electrode and the non-skin-side electrode.

According to the above-described absorbent article, on the side in the thickness direction where the liquid-impermeable sheet member (base sheet) is provided, it is possible to make it easier to suppress the contact between the conductive portion and the excrement. That is, it can makes it easier to prevent intruding the excrement from two widthwise sides of the liquid-impermeable sheet member and reaching it the conductive portion. Accordingly, the influence of the conductive portion and the excrement can be easily limited to the one side in the thickness direction (the side where the liquid-impermeable sheet member is not provided).

In such an absorbent article, it is desirable that a liquid-impermeable region is provided in at least a part of each of the skin-side electrode and the non-skin-side electrode, on a side in the thickness direction which is opposite to a side where the liquid-impermeable sheet member is provided.

According to the above-described absorbent article, for each electrode, it is possible to limit a conductive region. Accordingly, compared with the case where it is conductive between the electrodes across the entirety of the region, it is possible to keep low the amount of change in the capacitance value and the resistance value detected between the electrodes. Therefore, even when the amount of urine or the like absorbed by the absorbent body increases, the resistance value is less likely to exceed the lower limit and the capacitance value is less likely to exceed the upper limit, enabling accurate measurement.

In such an absorbent article, it is desirable that in the longitudinal direction, an area of the liquid-impermeable region on a front side with respect to a center is larger than an area of the liquid-impermeable region on a back side with respect to the center.

According to the above-described absorbent article, the front region in the longitudinal direction is a region where a large amount of urine is discharged and it makes it easier to conduct between the electrodes, and therefore increasing the area of the liquid-impermeable region makes it possible to keep low the amount of change in the capacitance and the resistance value detected between the electrodes. Therefore, even when the amount of urine absorbed by the absorbent body increases, the resistance value is less likely to exceed the lower limit and the capacitance value is less likely to exceed the upper limit, enabling accurate measurement.

In such an absorbent article, it is desirable that a thickness of the liquid-permeable sheet is thicker than thicknesses of the skin-side electrode and the non-skin-side electrode.

According to the above-described absorbent article, making the thickness of the liquid-impermeable sheet (top sheet) as thick as possible makes it easier to suppress a phenomenon such as a direct contact between the wearer's body (skin) and the skin-side electrode while the absorbent article is put on. Accordingly, it is possible to reduce the probability of erroneous detection of the resistance value and the capacitance value.

In such an absorbent article, it is desirable that end portions of the skin-side electrode and the non-skin-side electrode in the longitudinal direction of the skin-side electrode and the non-skin-side electrode are positioned outside at least one of a front end and a back end of the absorbent body in the longitudinal direction.

According to the above-described absorbent article, the skin-side electrode and the non-skin-side electrode both are arranged extending up to the longitudinal end region of the absorbent article. Therefore, at the time of connecting a detection device or the like to each electrode, it is possible to perform, in the longitudinal end region, work for connecting the device and each electrode. Accordingly, the detection device can be easily attached or detached, for example, even in a state in which the wearer puts on the absorbent article.

Further, a defecation/urination determination method in an absorbent article, the absorbent article including: a liquid-absorbent absorbent body; a liquid-permeable sheet that is arranged on a skin side in a thickness direction with respect to the absorbent body; and a liquid-impermeable sheet that is arranged on a non-skin side in the thickness direction with respect to the absorbent body, the method including: a skin-side capacitance detection process of detecting a magnitude of a capacitance by a skin-side electrode that is provided between the liquid-permeable sheet and the absorbent body; a non-skin-side capacitance detection process of detecting a magnitude of a capacitance by a non-skin-side electrode that is provided between the liquid-impermeable sheet and the absorbent body; and a determination process of determining whether excrement that has been excreted on the absorbent article is feces or urine based on an amount of change in the capacitance that has been detected in the skin-side capacitance detection process during a predetermined period and an amount of change in the capacitance that has been detected in the non-skin-side capacitance detection process during the predetermined period. At least one pair of the non-skin-side electrodes are provided spaced apart from each other in the width direction by a predetermined distance,
at least one pair of the skin-side electrodes are provided spaced apart from each other in the width direction by a distance that is different from the predetermined distance, and a space between the pair of skin-side electrodes in the width direction is larger than a space between the pair of non-skin-side electrodes in the width direction.

According to the above-described defecation/urination determination method, it is possible to detect changes in the skin-side capacitance value detected by the skin-side electrode and the non-skin-side capacitance value detected by the non-skin-side electrode during the predetermined period. Since the behaviors of changes in the skin-side capacitance value and the non-skin-side capacitance value are different between during urination and during defecation, measurement of the behaviors of the absorbent (2). these changes makes it possible to accurately determine whether the excrement is feces or urine.

In such a defecation/urination determination method, it is desirable that if the amount of change in the capacitance that has been detected by the skin-side electrode during the predetermined period is equal to or larger than a predetermined value, and if the amount of change in the capacitance that has been detected by the non-skin-side electrode during the predetermined period is equal to or larger than the predetermined value, it is determined that urine has been excreted, and that if the amount of change in the capacitance that has been detected by the skin-side electrode during the predetermined period is equal to or larger than the predetermined value, and if the amount of change in the capacitance that has been detected by the non-skin-side electrode during the predetermined period is less than the predetermined value, it is determined that feces has been excreted.

According to the above-described defecation/urination determination method, since urine permeates into the absorbent body, when urination is performed, both the skin-side electrode and the non-skin-side electrode are conducted, and the capacitance is more likely to be significantly changed. On the other hand, since the permeation of feces into the absorbent body is small, when defecation is performed, the skin-side electrode is conducted, and the capacitance is significantly changed. However, the non-skin-side electrode is less likely to be conducted, and the capacitance is less likely to be changed. Therefore, by utilizing such properties, it is possible to more accurately determine whether the excrement is feces or urine.

In such a defecation/urination determination method, it is desirable that concerning a timing at which the capacitance that has been detected by the skin-side electrode has changed during the predetermined period, and concerning a timing at which the capacitance that has been detected by the non-skin-side electrode has changed during the predetermined period, a difference between these timings is utilized as an additional index for determining urination.

According to the above-described defecation/urination determination method, in the case where urination has been performed, a predetermined time difference is generated between the time when urine has reached the skin-side electrode located on the skin side of the absorbent body and the time when the urine permeates into the absorbent body and has reached the non-skin-side electrode located on the non-skin side of the absorbent body. Therefore, by considering the difference in timing at which the capacitance detected by each electrode is changed, urination can be more accurately determined.

In such a defecation/urination determination method, it is desirable that concerning the timing at which the capacitance that has been detected by the skin-side electrode has changed during the predetermined period, and concerning the timing at which the capacitance that has been detected by the non-skin-side electrode has changed during the predetermined period, in a case where these timings are simultaneous, this case is determined as noise.

According to the above-described defecation/urination determination method, when urination is performed, the non-skin-side capacitance is supposed normally to start to change after a predetermined time has elapsed from the start of the change of the skin-side capacitance. Therefore, if the timing at which the skin-side capacitance is started to change and the timing at which the non-skin-side capacitance is started to change are the same, it is different from the behavior of normal urination. Therefore, this case is processed as noise, and erroneous determination can be suppressed.

In such a defecation/urination determination method, it is desirable that the method further comprises a skin-side resistance detection process of detecting a magnitude of a resistance value by the skin-side electrode, and concerning a degree of recovery during the predetermined period after the resistance value that has been detected by the skin-side electrode has changed, the degree is utilized as an additional index for determining urination.

According to the above-described defecation/urination determination method, the resistance value detected by the skin-side electrode when urination has been performed is likely to behave as follow: it decreases immediately after the urination, due to conducting between the skin-side electrodes, and it increases when a predetermined time elapses, due to de-conducting between the skin-side electrodes by absorption of the urine by the absorbent body. Therefore, urination can be more accurately determined by considering the degree of temporal change (degree of recovery) of the resistance value detected by the skin-side electrode.

In such a defecation/urination determination method, it is desirable that concerning a degree of recovery during the predetermined period after the capacitance value that has been detected by the skin-side electrode has changed, the degree is utilized as an additional index for determining defecation.

According to the above-described defecation/urination determination method, if defecation has been performed, the feces attached to the skin-side surface of the absorbent body continuously remains without being absorbed, and therefore the capacitance detected by the skin-side electrode is less likely to change even after the elapse of a predetermined time. Therefore, defecation can be more accurately determined by considering the degree of temporal change (degree of recovery) of the capacitance detected by the skin-side electrode.

### First Embodiment

As an example of an absorbent article according to a first embodiment, an absorbent pad 1 that absorbs excrement such as urine or feces will be described. The absorbent pad 1 is attached to the inside of a common disposable diaper (for example, a disposable diaper 101 described below).

### Basic Configuration

### Disposable Diaper 101

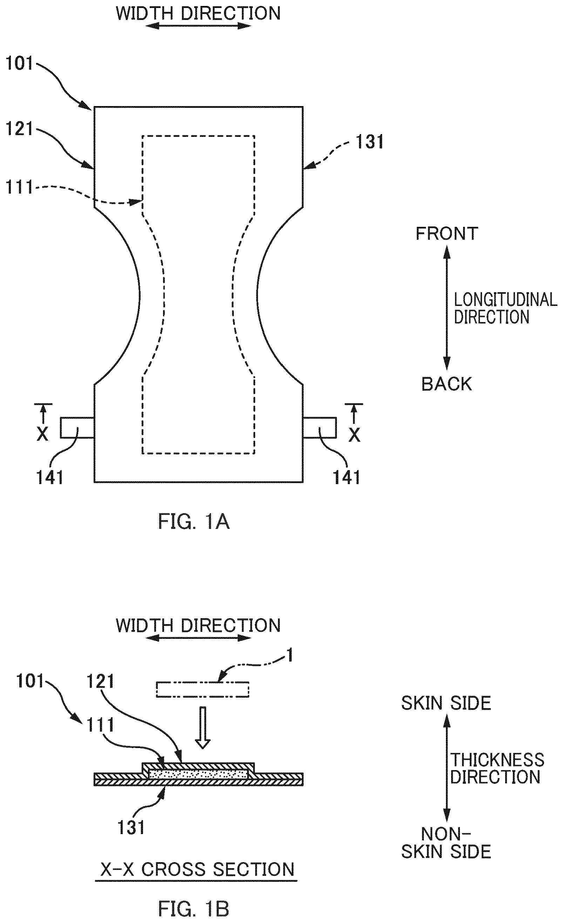

First, a disposable diaper 101 to which the absorbent pad 1 is attached (hereinafter, also simply referred to as a "diaper 101") will be described. FIG. 1A is a schematic plan view showing an unfolded state of the diaper 101. FIG. 1B is a schematic cross-sectional view showing a cross section taken along a line X-X in FIG. 1A. It should be noted that, although the diaper 101 shown in FIG. 1A is a so-called tape-type disposable diaper, the absorbent pad 1 can be used attached to another type of disposable diaper (for example, a pull-on disposable diaper) excluding the tape-type disposable diaper.

In FIGS. 1A and 1B, the diaper 101 has a longitudinal direction, a width direction, and a thickness direction that intersect with each other. The front side portion in the longitudinal direction is a portion positioned on the wearer's front side when the diaper 101 is put on, and the back side portion in the longitudinal direction is a portion positioned on the wearer's back side. Further, the skin side in the thickness direction is the side that comes into contact with the wearer's body (skin) while the diaper 101 is put on, and the non-skin side in the thickness direction is the side that does not come into contact with the wearer's body (skin).

The diaper 101 includes: an absorbent core 111 that is formed including a liquid absorbent material such as pulp fiber; a liquid-permeable top-surface sheet 121 that covers the absorbent core 111 from the skin side in the thickness direction; a liquid-impermeable back-surface sheet 131 that covers the absorbent core 111 from the non-skin side; and a pair of fastening tapes 141 that are provided in two widthwise end portions on the back side (back-side) in the longitudinal direction. Further, as shown in FIG. 1A, the shape of the diaper 101 in the unfolded state has a substantially hourglass shape having a longitudinal direction and a width direction. That is, the diaper 101 has a shape in which its longitudinal central portion is narrowed inward in the width direction. Then, the narrowed portion serves as a crotch portion and is applied to the wearer's crotch. the front portion in the longitudinal direction with respect to the crotch portion serves as a front panel and is applied to the wearer's lower abdomen. The back portion in the longitudinal direction with respect to the crotch portion serves as a back panel and is applied to the wearer's buttocks. The front panel and the back panel are fastened by the fastening tapes 141. In this way, the diaper 101 is put on the wearer's lower body.

The absorbent pad 1 according to the present embodiment is placed and attached to a skin-side surface of the top-surface sheet 121 of the diaper 101. Then, when the wearer puts on the diaper 101 that is in this state, the absorbent pad 1 is put on the wearer's lower body while being integrated with the diaper 101. It should be noted that, in some cases, a displacement-preventing adhesive portion formed of a hot-melt adhesive, or a male member of a hook-and-loop fastener, or the like may be provided on the non-skin-side surface of the absorbent pad 1 to fix the absorbent pad 1 so as not to relatively move from a state in which the absorbent pad 1 is placed on the diaper 101.

### Absorbent Pad 1

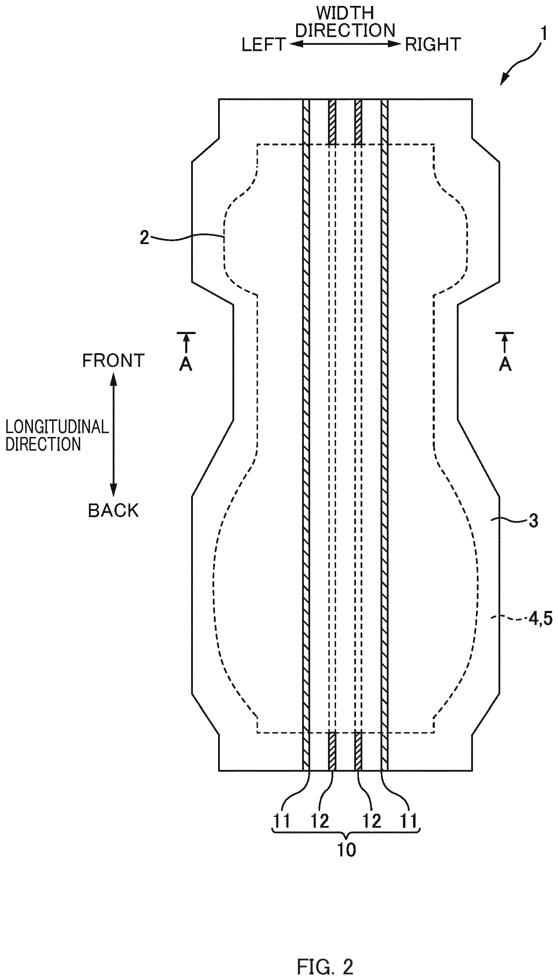

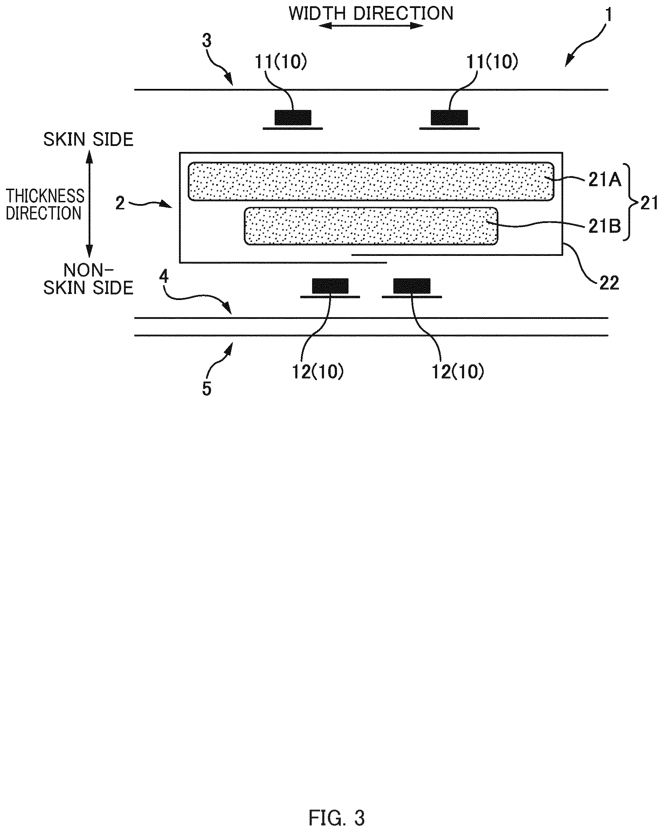

Next, the absorbent pad 1 will be described. FIG. 2 is a schematic plan view of an absorbent pad 1. FIG. 3 is a schematic cross-sectional view showing a cross section taken along a line A-A in FIG. 2. As shown in FIGS. 2 and 3, the absorbent pad 1 has a longitudinal direction, a width direction, and a thickness direction as three directions that are orthogonal to each other. These directions respectively correspond to the longitudinal direction, the width direction, and the thickness direction in FIG. 1.

The absorbent pad 1 is an absorbent article which is used being attached to the skin-side surface of the diaper 101, and as shown in FIG. 2, has a substantially hourglass shape in which the longitudinal central portion is narrowed inward in the width direction. The absorbent pad 1 includes: an absorbent body 2; a top sheet 3 that is arranged on the skin side in the thickness direction with respect to the absorbent body 2; a leak-proof sheet 4 that is arranged on the non-skin side in the thickness direction with respect to the absorbent body 2; and a back sheet 5 that is arranged on the non-skin side of the leak-proof sheet 4. Further, the absorbent pad 1 is provided with electrodes 10 for detecting urination and defecation. The components that are adjacent to each other in the thickness direction are joined to each other with a joining material such as a hot-melt adhesive.

The absorbent body 2 is a liquid-absorbent member having an absorbent core 21 and a core-wrapping sheet 22. The absorbent core 21 includes a polymer absorbent (absorbent polymer: super absorbent polymer, hereinafter, also referred to as "SAP") and liquid-absorbent fibers such as pulp fibers, and has a substantially hourglass shape in which the longitudinal central portion is narrowed inward in the width direction, similar to the absorbent pad 1 (see FIG. 2). In the present embodiment, as shown in FIG. 3, the absorbent core 21 has a two-layer structure including a skin-side core layer 21A that is provided on the skin side in the thickness direction and a non-skin-side core layer 21B that is overlaid on the non-skin side of the skin-side core layer 21A. However, the absorbent core 21 may have a single-layer structure or a multilayer structure having three or more core layers. The core-wrapping sheet 22 is a liquid-permeable sheet member that covers the absorbent core 21, and is formed of, for example, tissue paper or the like.

It should be noted that the configuration of the absorbent body 2 is not limited to the above, and examples thereof include an SAP sheet in which an SAP layer is attached to a hydrophilic sheet, an air-laid sheet in which liquid-absorbent fibers are formed into a sheet by an air-laid method, and the like.

The top sheet 3 is a liquid-permeable sheet member (liquid-permeable sheet) that is arranged farthest on the skin side in the thickness direction of the absorbent pad 1, and is a member that comes into direct contact with the wearer's skin while the absorbent pad 1 is put on. The top sheet 3 receives urine and feces which have been excreted from a human body, and rapidly absorbs the urine and feces in the thickness direction, introducing the urine and feces to the absorbent body 2 (absorbent core 21). As for the top sheet 3, a sheet larger than the plan shape of the absorbent body 2 is used. As the sheet member constituting the top sheet 3 of the present embodiment, an air-through nonwoven fabric, a spunbond nonwoven fabric, and the like can be exemplified.

The leak-proof sheet 4 is a liquid-impermeable sheet member (liquid-impermeable sheet) that is arranged on the non-skin side in the thickness direction of the absorbent pad 1 with respect to the absorbent body 2. As for the leak-proof sheet 4, a sheet larger than the plan shape of the absorbent body 2 is used, and providing the leak-proof sheet 4 prevents the liquid such as urine that has been absorbed by the absorbent body 2 from permeating into the clothing side of the wearer (non-skin side). As the sheet member constituting the leak-proof sheet 4 of the present embodiment, resin films made of polyethylene or polypropylene or the like can be exemplified.

The back sheet 5 is a member (exterior sheet) that is arranged on the non-skin side of the leak-proof sheet 4 in the thickness direction of the absorbent pad 1, and constitutes the exterior of the absorbent pad 1. The back sheet 5 has substantially the same size as the leak-proof sheet 4. Examples of the sheet member constituting the back sheet 5 of the present embodiment include an air-through nonwoven fabric and the like. Further, on the non-skin-side surface of the back sheet 5, a displacement-preventing adhesive portion or the like which is for attaching and fixing the absorbent pad 1 to the skin-side surface of the diaper 101 may be provided.

Further, although not shown in FIGS. 2 and 3, a pair of leak-proof wall portions which suppress lateral leakage of urine or the like may be provided on the skin side of the absorbent body 2 in the thickness direction and in two widthwise end portions of the absorbent body 2. Since the leak-proof wall portions are well-known, the detailed description thereof will be omitted.

### Electrode 10

The electrode 10 is a detection portion for detecting excretion by coming into contact with excrement (e.g., urine or feces) that has been excreted from the wearer. In the thickness direction, the electrode 10 of the present embodiment includes: skin-side electrodes 11 that are arranged between the top sheet (liquid-permeable sheet) 3 and the absorbent body 2; and non-skin-side electrodes 12 that are arranged between the leak-proof sheet (liquid-impermeable sheet) 4 and the absorbent body 2 (see FIG. 3). The skin-side electrodes 11 are strip-shaped electrodes that extend along the longitudinal direction and that are provided in a pair with a predetermined space in the width direction (see FIG. 2). Similarly, the non-skin-side electrodes 12 are strip-shaped electrodes that extend along the longitudinal direction and that are provided in a pair with a predetermined space in the width direction (with a space different from the space between the skin-side electrodes 11). However, the electrodes 11 and 12 each may be provided in multiple pairs.

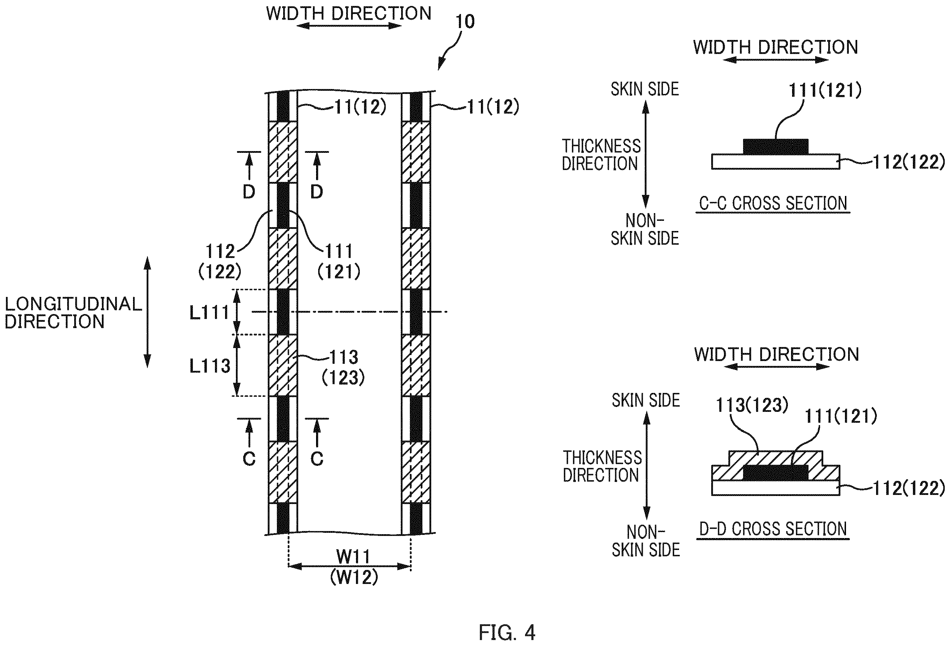

FIG. 4 is a schematic plan view and a cross-sectional view illustrating an example of a configuration of the pair of skin-side electrodes 11 and 11. The skin-side electrode 11 includes a conductive portion 111, a base sheet 112, and covering portions 113. The conductive portion 111 is formed by continuously applying a conductive ink in a strip shape across the overall longitudinal length of the absorbent pad 1. In the present embodiment, as shown in a cross-sectional view taken along C-C in FIG. 4, the conductive portion 111 is formed by applying a conductive ink to one-side surface of the base sheet 112 in the thickness direction (skin-side surface in FIG. 4). The conductive ink is prepared by kneading a binder, a conductive metal powder, and a filler. As such a binder, it is possible to use polyvinyl chloride-based resin, polyacrylic resin, epoxy-based resin, polyester-based resin, polyacrylic urethane-based resin, polyolefin-based resin, polyurethane-based resin, phenol-based resin, and the like. As such a conductive metal powder, it is possible to use silver, gold, copper, nickel, aluminum, conductive carbon, or the like. Such a filler includes a viscosity adjusting agent, a dispersing agent, and the like. It should be noted that the configuration of the conductive ink is not limited to these examples, but it is desirable that the conductive ink is formed of a substance which makes electricity easily flow and which has as a low resistance as possible from the viewpoint of detection accuracy.

The base sheet 112 is a liquid-impermeable sheet member having flexibility, and is formed of a material having a lower conductivity than the conductive portion 111. As a material of the base sheet 112, it is possible to use, for example, a biaxially stretched film made of polypropylene, polyethylene, polyvinyl chloride, polyester, polyamide, polyimide, polyamide imide, polycarbonate, or polystyrene. In the present embodiment, by performing annealing treatment (heat treatment) on a PET sheet having a thickness of approximately 25 µm, flexibility is imparted to the base sheet 112 (a liquid-impermeable sheet member). This makes the wearer less likely to have discomfort while the absorbent pad 1 is put on.

The base sheet 112 is arranged overlaid on the one side of the conductive portion 111 in the thickness direction, and therefore the influence of the contact between the conductive portion 111 and excrement can be limited to the one side in the thickness direction. That is, on the one side in the thickness direction, the contact of the conductive portion 111 with excrement is limited by the base sheet 112, and, on the other side in the thickness direction, the conductive portion 111 is exposed and thus easily comes into contact with the excrement. Therefore, if the conductive portion 111 is provided on the skin side of the base sheet 112 (liquid-impermeable sheet member) (see FIG. 4), it is possible to make it easier to detect in the skin-side electrode 11 the influence of the excrement that has attached to the top sheet 3 (liquid-permeable sheet) side. On the other hand, if the conductive portion 111 is provided on the non-skin side of the base sheet 112 (not shown), it is possible to make it easier to detect in the skin-side electrode 11 the influence of the excrement that has attached to the skin-side surface of the absorbent body 2. In this manner, by limiting the surface where the conductive portion 111 is exposed in the skin-side electrode 11 to only the one side in the thickness direction, it is possible to select whether to detect the influence of the top sheet 3 (liquid-permeable sheet) side or to detect the influence of the absorbent body 2 side.

Further, two widthwise ends of the base sheet 112 are positioned outside the two widthwise ends of the conductive portions 111. That is, the width of the base sheet 112 is larger than the width of the conductive portion 111, and the conductive portions 111 are not arranged in two widthwise end portions of the base sheet 112. With such a configuration, it is possible to make it easier to suppress the contact between the conductive portion 111 and the excrement on the side in the thickness direction where the base sheet 112 is provided. For example, it is possible to prevent intruding the excrement from two widthwise sides of the base sheet 112 and reaching it to the conductive portion 111. That is, the influence of the contact between the conductive portion 111 and the excrement can be limited to only the one side in the thickness direction (the side where the base sheet 112 is not provided).

The covering portions 113 are each a liquid-impermeable member that covers at least a partial region of the conductive portion 111 from the side where the base sheet 112 is not provided, in the thickness direction (in FIG. 4, the skin side), as shown in the cross section taken along D-D in FIG. 4. For example, the covering portion 113 can be formed of a film of polyethylene (PE), polypropylene (PP), polyethylene terephthalate (PE), or the like. The above-described covering portions 113 each form a liquid-impermeable region in at least a portion of the skin-side electrode 11, on the side in the thickness direction which is opposite to the side where the base sheet 112 (liquid-impermeable sheet member) is provided. In the liquid-impermeable region, the contact of moisture such as urine with the conductive portion 111 is suppressed. Therefore, in the skin-side electrode 11, a current between the pair of skin-side electrodes 11 and 11 is less likely to flow in the liquid-impermeable region in which the conductive portion 111 is covered with the covering portion 113 on the skin side. On the other hand, in the skin-side electrode 11, in the region where the conductive portion 111 is exposed without being covered with the covering portion 113 (hereinafter, also referred to as a "liquid-permeable region"), the moisture such as urine between the pair of skin-side electrodes 11 and 11 conducts between the electrodes 11, making the current easier to flow.

In the skin-side electrode 11 of the present embodiment, as shown in FIG. 4, since the liquid-permeable region and the liquid-impermeable region are alternately located in the longitudinal direction, conductive regions are limited. If the liquid-impermeable region is not provided in the skin-side electrode 11, the entire region between the pair of skin-side electrodes 11 and 11 can be conducted, making the current easily flow. That is, it makes it more likely to decrease the resistance value detected between the electrodes, and to increase the capacitance value detected between the electrodes. In this case, as the amount of urine or the like absorbed by the absorbent body 2 increases, the detectable resistance becomes more likely to exceed the lower limit value and the capacitance becomes more likely to exceed the upper limit value, causing a risk that accurate measurement is not possible. In contrast, in the present embodiment, since the liquid-impermeable regions are provided to partially limit the current flowing between the pair of skin-side electrodes 11 and 11, the resistance becomes less likely to exceed the lower limit value and the capacitance becomes less likely to exceed the upper limit value. This makes it possible to perform accurate measurements even when the amount of absorbed urine or the like increases.

Further, letting the longitudinal length of the liquid-permeable region be L111, letting the longitudinal length of the liquid-impermeable region be L113, it is desirable that the sum of the lengths L113 in the region on the front side in the longitudinal direction with respect to the longitudinal center is longer than the sum of the lengths of L113 in the region on the back side in the longitudinal direction with respect to the longitudinal center. In other words, it is desirable that the area of the liquid-impermeable regions on the front side with respect to the longitudinal center is larger than the area of the liquid-impermeable regions on the back side with respect to the longitudinal center. In the region on the front side in the longitudinal direction, a large amount of urine is more likely to be discharged, making it easier to conduct between the electrodes 11 and 11. Therefore, by increasing the area of the liquid-impermeable region in the region, it is possible to make the resistance value less likely to exceed the lower limit and to make the capacitance value less likely to exceed the upper limit, even when the amount of urine absorbed increases. This can make more accurate measurements easier.

Further, the space between the pair of skin-side electrodes 11 and 11 in the width direction is defined as W11. W11 refers to the distance between the inner ends of the pair of conductive portions 111 and 111 in the width direction (see FIG. 4). In the present embodiment, the skin-side electrodes 11 are configured so that the space W11 is 40 mm or larger. If the space W11 is less than 40 mm, the current easily excessively flows between the electrodes and causes noise and the like, and erroneous detection is likely to occur. Further, in the case where the space W11 between the electrodes is 40 mm or larger, it is possible to make it easier to appropriately adjust the upper limit and the lower limit of the capacitance value and the resistance value detected between the electrodes.

The configuration of the pair of non-skin-side electrodes 12 and 12 is also substantially the same as the configuration of the pair of skin-side electrodes 11 and 11 shown in FIG. 4. That is, the non-skin-side electrode 12 includes a conductive portion 121, a base sheet 122 (a liquid-impermeable sheet member), and a covering portion 123. Further, for the same reason as the skin-side electrode 11, it is desirable that the space W12 between the pair of non-skin-side electrodes 12 and 12 in the width direction is 40 mm or larger. However, in the present embodiment, the widthwise space W12 between the pair of non-skin-side electrodes 12 and 12 and the widthwise space W11 between the pair of skin-side electrodes 11 and 11 are different from each other. More specifically, the space W12 is smaller than the space W11 (W11 > W12, see FIG. 2, and the like). In the case of urination, the skin-side surface of the absorbent body 21 is more likely to get wet, and therefore widening the space W11 between the skin-side electrodes 11 and 11, which urine easily reaches, makes it possible to relatively suppress an increase in the capacitance value and a decrease in the resistance value with respect to the non-skin-side electrodes 12 and 12. Accordingly, this makes it possible to suppress that the determination based on combination of the changes of the detected values by both of the electrodes 11 and 12 (the details will be described later) cannot be used.

Further, in the present embodiment, it is desirable that the thickness of the top sheet 3 (liquid-permeable sheet) is thicker than the thickness of each of the electrodes 11 and 12. Specifically, it is desirable that the thickness of the base sheets 111 and 112 respectively constituting the electrodes 11 and 12 is approximately 25 µm, whereas the thickness of the top sheet 3 is approximately 0.5 mm to 1.5 mm. While the absorbent pad 1 is put on, the top sheet 3 is positioned between the wearer's body (skin) and the skin-side electrodes 11. Therefore, making the thickness of the top sheet 3 as thick as possible makes it easier to suppress the influence of contact of the wearer's body with the skin-side electrodes 11, the influence of pressing the wearer's body against the skin-side electrodes 11, and the like. Accordingly, it is possible to reduce the probability of erroneous detection of the resistance value and the capacitance in the electrodes 11 and 12.

### Method for Detecting Excrement

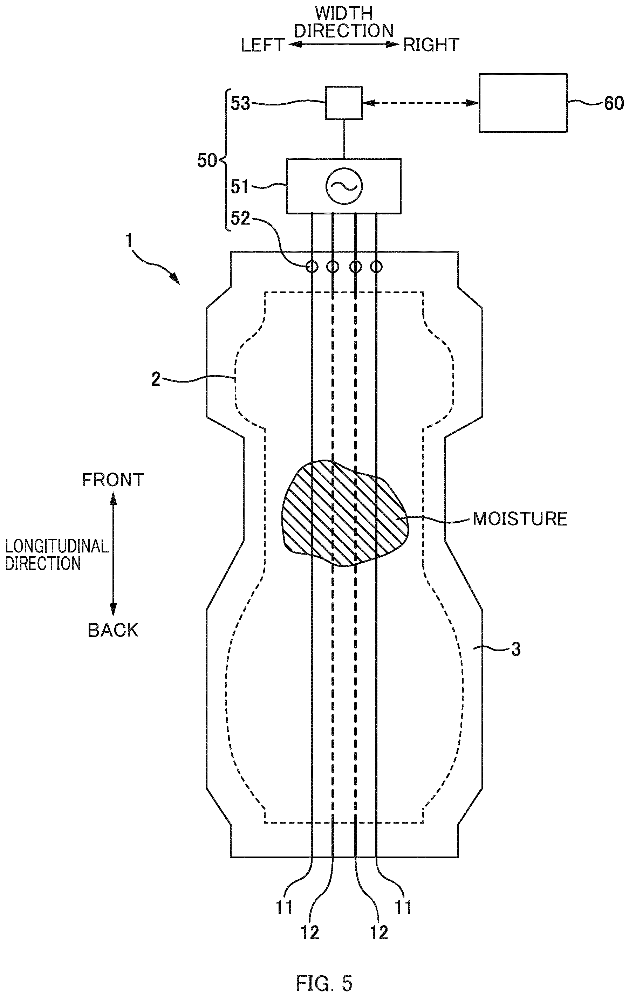

A method for detecting excrement using the absorbent pad 1 will be described. FIG. 5 is a conceptual diagram of an excrement detection circuit using the absorbent pad 1. In the case of detecting excrement, the absorbent pad 1 is connected to a detection device 50 and an information processing apparatus 60, and various data are measured.

The detection device 50 is a device that applies an AC current to the electrodes 10 (the skin-side electrodes 11 and the non-skin-side electrodes 12) of the absorbent pad 1 and measures the capacitance value and the resistance value each of which has been detected between the pair of skin-side electrodes 11 and 11 and between the pair of non-skin-side electrodes 12 and 12. The detection device 50 includes a main body unit 51, connection units 52, and a data transmitting and receiving unit 53.

The main body unit 51 includes at least: a power supply unit (a battery, a cell, or the like) for applying a current to the electrodes 10; and a measurement unit for measuring a capacitance value and a resistance value detected by the electrodes 10 (neither shown). Further, the main body unit 51 may include a ground or the like in order to discharge electricity charged in the electrodes 10. It should be noted that, in the case of applying a DC current to the electrodes 10, it is desirable that there is provided a polarity reversal circuit for applying currents having different polarities to the pair of left and right electrodes 11 and 11 (12 and 12).

The connection units 52 are each a connector that connects the main body unit 51 and the electrodes 11 and 12 of the absorbent pad 1. Through the connection units 52, a current is applied to the electrodes 10, and a capacitance value and a resistance value between the electrodes are detected. In the present embodiment, the space W11 between the pair of skin-side electrodes 11 and 11 in the width direction is different from the space W12 between the pair of non-skin-side electrodes 12 and 12 in the width direction, and therefore each set of the electrodes 11 and 12 is arranged at a different position in the width direction. That is, since the positions of the electrodes do not overlap with respect to the width direction, as in FIG. 5, the connection units 52 are easily connected to each of the electrodes 11 and 12, and the detection device 50 can be easily attached.

Further, as shown in FIGS. 3 and 4, in the present embodiment, in both of the skin-side electrode 11 and the non-skin-side electrode 12, the base sheets 112 and 122 are provided on the same side in the thickness direction. In other words, the conductive portions 111 and 121 are in a state of being exposed on the same side in the thickness direction. This enables each of the connection units 52 to connect to each of the electrodes 11 and 12 on the same side in the thickness direction, making it easier to attach the detection device 50.

Further, it is desirable that in the longitudinal direction, the end portions of the skin-side electrodes 11 and the non-skin-side electrodes 12 are positioned outside at least one of the front end and the back end of the absorbent body 2. In the present embodiment, as shown in FIGS. 2 and 5, in the longitudinal direction, the front end portions of the skin-side electrodes 11 and the non-skin-side electrodes 12 are positioned outside the front end of the absorbent body 2. That is, both the skin-side electrodes 11 and the non-skin-side electrodes 12 are arranged extending up to the front end region of the absorbent pad 1 in the longitudinal direction. Therefore, in the front end region in the longitudinal direction, it is possible to perform work for connecting each of the electrodes 11 and 12 and the connection units 52. Since the connection position is in the front end portion in the longitudinal direction, the detection device 50 can be easily attached or detached even in a state in which the wearer puts on the absorbent pad 1.

The information processing apparatus 60 is configured by, for example, a workstation, a personal computer, or the like, and has a function as a so-called server. In the present embodiment, the information processing apparatus 60 stores various data transmitted from the detection device 50, and detects that excretion has been performed, based on the data, and performs a process of determining whether excrement is feces or urine and determining the amount of excrement (for example, an amount of urine excreted). Various processes performed by the information processing apparatus 60 will be described later.

It should be noted that by communicably connecting the information processing apparatus 60 to the plurality of different absorbent pads 1 (and the detection device 50), it is possible to detect the excretion state of a plurality of users (the wearers of the absorbent pads 1). Further, by connection with an external terminal such as a smartphone, it is possible to transmit and receive information on the excretion state to and from the terminal. Further, a configuration is also acceptable in which the detection device 50 has the function of the information processing apparatus 60 and performs a process of detecting excretion or making various determinations by itself.

Further, a configuration is also acceptable in which the information processing apparatus 60 individually acquires the detection results of urination and defecation and performs various estimations from the individually acquired detection results. In the present embodiment, on the skin surface side of the absorbent body 2, the capacitance value and the electrical resistance value are detected by the skin-side electrode 11 based on the excrement attached to the top sheet 3, and these electrical characteristic values are mainly used for estimating the presence or absence of defecation. On the other hand, on the non-skin surface side of the absorbent body 2, the capacitance value and the electrical resistance value are detected by the non-skin-side electrode 12 based on the excrement absorbed in the absorbent core 21, and these electrical characteristic values are mainly used for estimating the presence or absence of urination. That is, the electrical characteristic values mainly used for defecation determination and the electrical characteristic values mainly used for urination determination are individually detected and individually transmitted to the information processing apparatus 60. In this case, it can be said that the information processing apparatus 60 individually acquires defecation information, which is information related to defecation of the user, and urination information, which is information related to urination of the user.

That is, structures for measuring capacitance and electrical resistance (electrodes) are located at different positions of the absorbent article, and the electrical characteristic values (capacitance value and electrical resistance value) detected by each of the structures (electrodes) are associated with information indicating by which structure the information is detected, and the resultants are individually transmitted to the information processing apparatus 60. The information processing apparatus 60 that has acquired the information may estimate defecation and urination.

If the information processing apparatus 60 individually acquires information as described above, this can make it easier to perform an appropriate process according to each case of urination and defecation. For example, if defecation information is acquired, an alert prompting a caregiver or the like to immediately change the absorbent pad 1 is issued. On the other hand, if urination information is acquired, an alert is issued when that the absorption capacity reaches the limit is determined according to the absorption capacity (capacity capable of absorbing urine) of the absorbent core 21. This makes the caregiver easier to recognize an appropriate timing for changing the absorbent pad 1, and this makes it possible to reduce the burden of confirmation work for changing the absorbent pad 1 and of a cleaning process when excretion leakage occurs.

Further, a configuration is also acceptable in which the electrical characteristic values (capacitance value and electrical resistance value) detected by each structure (electrode) is periodically transmitted to the information processing apparatus 60 and the information processing apparatus 60 performs various estimations based on changes in the electrical characteristic values. For example, the information processing apparatus 60 can enhance the accuracy of estimation of defecation and urination by storing periodically-acquired information, generating an excretion history for each user, and using the excretion history. In addition, by monitoring the changes of the electrical characteristic values, it is possible to estimate items such as the amount of excrement, and the feces quality when the excrement is feces.

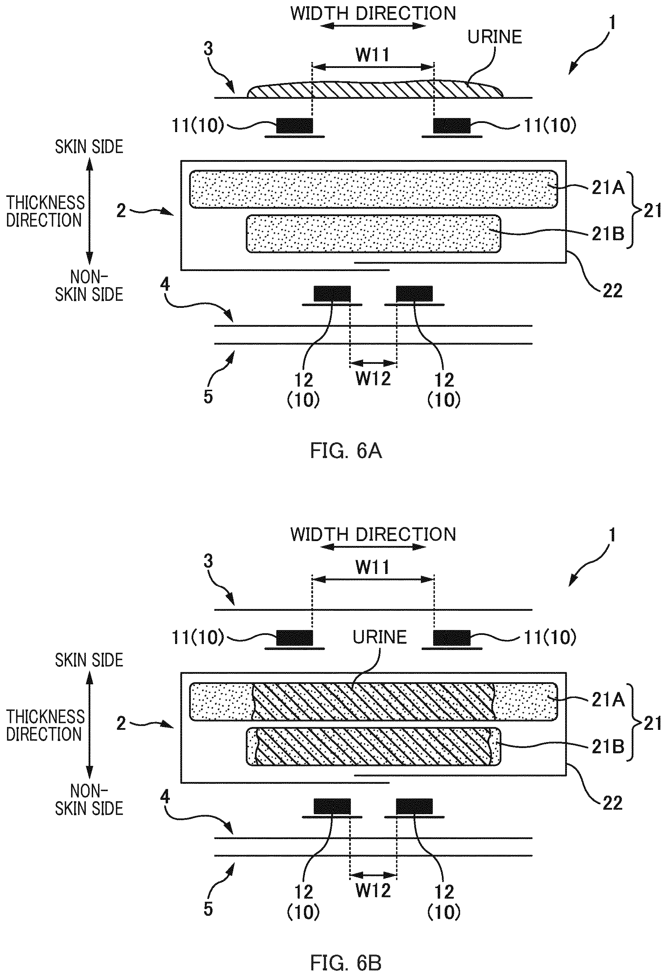

Next, the principle of detecting urine using the absorbent pad 1 will be described. FIGS. 6A and 6B are diagrams illustrating the principle of detecting urine when urine is excreted. FIG. 6A is a schematic cross-sectional view of the absorbent pad 1 shown in FIG. 3, and shows a state immediately after urine is excreted on the skin-side surface of the absorbent pad 1. Further, FIG. 6B shows a state after a predetermined time (for example, 30 seconds) has elapsed from the state in FIG. 6A.

When urination is performed in a state where the wearer puts on the absorbent pad 1, the excreted urine is first attached to the skin-side surface of the top sheet 3, then permeates into the top sheet 3 from the skin side to the non-skin side in the thickness direction, and is moved to the absorbent body 2. At this time, there is a case where the skin-side electrode 11 arranged between the top sheet 3 and the absorbent body 2 comes into contact with urine depending on conditions such as the amount of urine excreted. In FIG. 6A, urine is discharged so as to straddle the pair of skin-side electrodes 11 and 11 which are located apart from each other with the space W11 in the width direction. Therefore, the moisture contained in the urine conducts between the conductive portions 111 and 111, and the current is more likely to flow between the skin-side electrodes 11 and 11 compared with a state before urination (that is, a state in which the skin-side electrodes 11 and 11 are insulated by drying).

Therefore, before the moisture in the urine conducts between the skin-side electrodes 11 and 11, a resistance value R11 detected between the pair of skin-side electrodes 11 and 11 is great (the current is less likely to flow), and when the moisture in the urine conducts between the skin-side electrodes 11 and 11, the resistance value R11 decreases (the current is more likely to flow). On the other hand, before the moisture in the urine conducts between the skin-side electrodes 11 and 11, a capacitance value C11 detected between the pair of skin-side electrodes 11 and 11 is small (the electric charges are less likely to be accumulated), and when the moisture in the urine conducts between the skin-side electrodes 11 and 11, the capacitance value C11 increases (the electric charges are more likely to be accumulated).

When a predetermined time elapses after urine is discharged, the urine that has permeated into the top sheet 3 is absorbed by the absorbent body 2, and permeates into the inside of the absorbent body 2 from the skin side to the non-skin side. At this time, there is a case where the non-skin-side electrode 12 arranged between the absorbent body 2 and the leak-proof sheet 4 comes into contact with the urine. In FIG. 6B, the urine absorbed by the absorbent body 2 (indicated by hatched portions in FIG. 6B) is diffused so as to straddle a pair of non-skin-side electrodes 12 and 12 which are located apart from each other with the space W12 in the width direction. Therefore, the moisture contained in the urine conducts the conductive portions 121 and 121 of the non-skin-side electrodes 12, and the current is more likely to flow between the non-skin-side electrodes 12 and 12 compared with a state before urination (a state in which the non-skin-side electrodes 12 and 12 are insulated by drying).

The behavior of a resistance value R12 and a capacitance value C12 which have been detected by the non-skin-side electrodes 12 is substantially the same as that of the skin-side electrode 11. That is, before the moisture in the urine conducts between the non-skin-side electrodes 12 and 12, the resistance value R12 detected between the pair of non-skin-side electrodes 12 and 12 is great (the current is less likely to flow), and when the moisture in the urine conducts between the non-skin-side electrodes 12 and 12, the resistance value R12 decreases (the current is more likely to flow). On the other hand, before the moisture in the urine conducts between the non-skin-side electrodes 12 and 12, the capacitance value C12 detected between the non-skin-side electrodes 12 and 12 is small (the electric charges are less likely to be accumulated), and when the moisture in the urine conducts between the non-skin-side electrodes 12 and 12, the capacitance value C12 increases (the electric charges are easily accumulated).

It should be noted that, in the present embodiment, both the skin-side electrode 11 and the non-skin-side electrode 12 are provided with liquid-impermeable base sheets 112 and 122, on the non-skin side in the thickness direction. Both the conductive portions 111 and 121 are in a state of being exposed on the skin side. Therefore, this makes excrement such as urine excreted on the skin side more likely to come into contact with the conductive portions 111 and 121 of the electrodes 11 and 12, enabling to make it easier to detect the excrement.

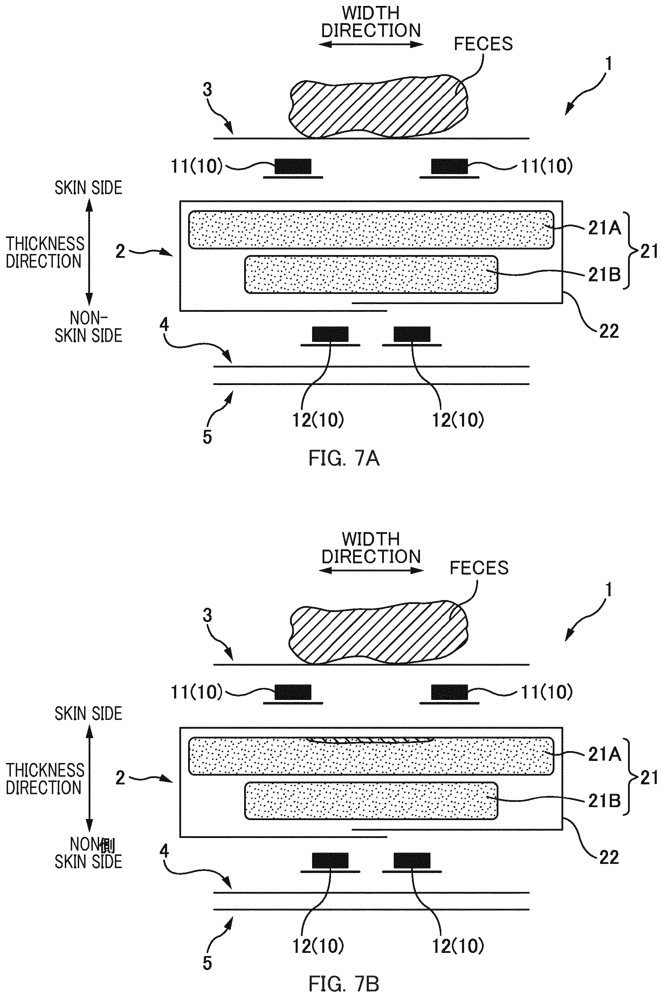

Next, the principle of detecting feces using the absorbent pad 1 will be described. FIGS. 7A and 7B are diagrams illustrating the principle of detecting feces when feces is excreted. FIG. 7A is a schematic cross-sectional view of the absorbent pad 1 shown in FIG. 3, and shows a state immediately after feces is excreted on the skin-side surface of the absorbent pad 1. Further, FIG. 7B shows a state after a predetermined time (for example, 30 seconds) has elapsed from the state in FIG. 7A.

When defecation is performed in a state in which the wearer puts on the absorbent pad 1, the excreted feces is attached to the skin-side surface of the top sheet 3. At this time, there is a case where moisture contained in the feces permeate into the top sheet 3 and come into contact with the skin-side electrode 11 arranged between the top sheet 3 and the absorbent body 2. In FIG. 7A, the feces is discharged so as to straddle the pair of skin-side electrodes 11 and 11 in the width direction. Therefore, the moisture contained in the feces conducts between the conductive portions 111 and 111, and the current is more likely to flow between the skin-side electrodes 11 and 11 compared with a state before defecation (that is, a state in which the skin-side electrodes 11 and 11 are insulated by drying).

Therefore, before the moisture in the feces conducts between the skin-side electrodes 11 and 11, the resistance value R11 detected between the pair of skin-side electrodes 11 and 11 is great (the current is less likely to flow), and when the moisture in the feces conducts between the skin-side electrodes 11 and 11, the resistance value R11 decreases (the current is more likely to flow). On the other hand, before the moisture in the feces conducts between the skin-side electrodes 11 and 11, the capacitance value C11 detected between the pair of skin-side electrodes 11 and 11 is small (the electric charges are less likely to be accumulated), and when the moisture in the feces conducts between the skin-side electrodes 11 and 11, the capacitance value C11 increases (the electric charges are more likely to accumulated).

On the other hand, the amount of moisture contained in the feces is small compared with the amount of moisture contained in the urine, and the feces is less likely to permeate into the absorbent body 2, and therefore even after a predetermined time has elapsed since the feces was discharged, the moisture contained in the feces is less likely to reach the non-skin side of the absorbent body 2. That is, there is a low possibility that the moisture in the feces comes into contact with the non-skin-side electrodes 12 arranged between the absorbent body 2 and the leak-proof sheet 4. In FIG. 7B, the moisture contained in the feces remains on the skin-side surface portion of the absorbent body 2 (indicated by hatched portions in FIG. 7B) and does not come into contact with the non-skin-side electrodes 12 and 12. Therefore, the conductive portions 121 and 121 of the non-skin-side electrodes 12 are not conducted, and the current is less likely to flow between the non-skin-side electrodes 12 and 12.

That is, the non-skin-side electrode 12 is less likely to be affected by the moisture in the feces, and the state between the electrodes is less likely to be changed before and after defecation. Therefore, the resistance value R12 detected between the pair of non-skin-side electrodes 12 and 12 is less likely to be changed before and after defecation. Similarly, the capacitance value C12 detected between the pair of non-skin-side electrodes 12 and 12 is less likely to be changed before and after defecation.

As described above, when defecation is performed, the resistance value R11 and the capacitance value C11 detected by the skin-side electrodes 11 are changed, but the resistance value R12 and the capacitance value C12 detected by the non-skin-side electrodes 12 are substantially not changed. Therefore, by monitoring the temporal changes in the resistance values and the capacitance values detected by the skin-side electrodes 11 and the non-skin-side electrodes 12, it is possible to accurately determine whether the excrement is feces or urine.

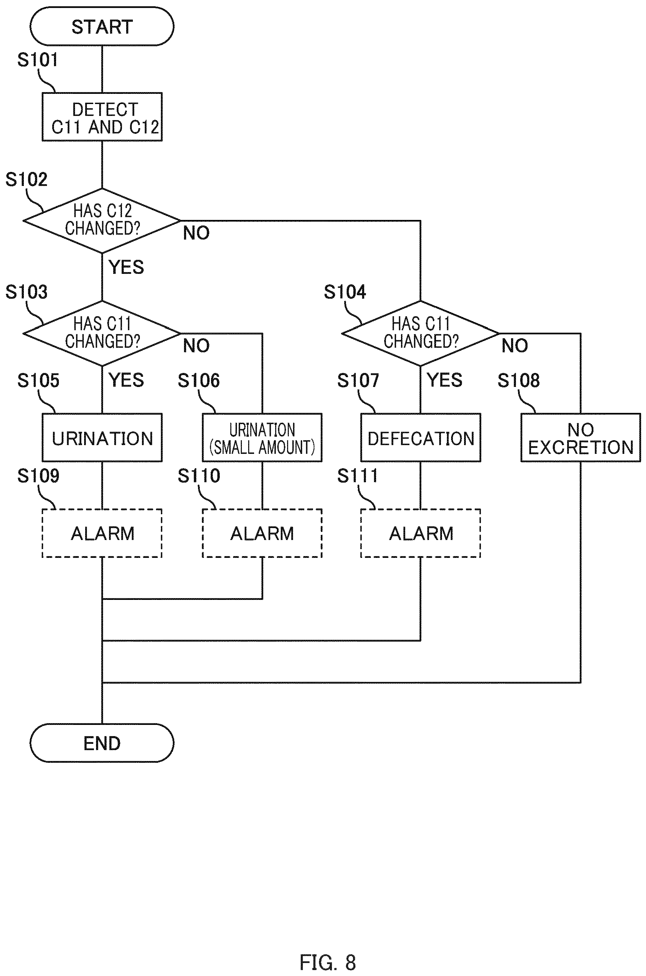

FIG. 8 is a flow chart showing an example of a defecation/urination determination process using the absorbent pad 1. Various processes in the defecation/urination determination are mainly performed by the information processing apparatus 60. Hereinafter, for the simplification of description, description will be made on the assumption that various processes are performed by the detection device 50.

When the detection of excrement is started, the detection device 50 intermittently applies a current to each of the electrodes 11 and 12 of the absorbent pad 1. Then, the following detection processes are performed (S101): a non-skin-side capacitance detection process of detecting the magnitude of the capacitance value C12 between the non-skin-side electrodes 12 and 12; and a skin-side capacitance detection process of detecting the magnitude of the capacitance value C11 between the skin-side electrodes 11 and 11.

Next, the detection device 50 (information processing apparatus 60) determines the amount of change in the capacitance value C12 during a predetermined period (for example, a measurement period of approximately 1 to 30 seconds) based on the detection result of the non-skin-side capacitance detection process (S102). As a result, if the amount of change in the capacitance value C12 is equal to or larger than a predetermined magnitude (Yes in S102), the process proceeds to step S103. The case where the amount of change in the capacitance value C12 is equal to or larger than the predetermined magnitude means a state in which moisture such as urine reaches between the pair of non-skin-side electrodes 12 and 12 and conducts between the electrodes as described in FIG. 6B. That is, this indicates that there is a high possibility that urination has been performed.

On the other hand, if the amount of change in the capacitance value C12 is less than the predetermined magnitude (No in S102), the process proceeds to step S104. The case where the amount of change in the capacitance value C12 is not more than or is less than the predetermined magnitude means a state in which moisture does not reach between the pair of non-skin-side electrodes 12 and 12 and does not make conduct between the electrodes as described in FIG. 7B. That is, this indicates that a possibility that defecation has been performed or that neither urine nor feces has been excreted is high.

In step S103, the detection device 50 determines the amount of change in the capacitance value C11 during the predetermined period based on the detection result of the skin-side capacitance detection process (S103). As a result, if the amount of change in the capacitance value C11 is equal to or larger than the predetermined magnitude (Yes in S103), the process proceeds to step S105. The case where the amount of change in the capacitance value C11 is equal to or larger than the predetermined magnitude means a state in which moisture such as urine reaches between the pair of skin-side electrodes 11 and 11 and conducts between the electrodes as described in FIG. 6A. That is, it means a state in which the pair of non-skin-side electrodes 12 and 12 are conducted therebetween in S102 and the pair of skin-side electrodes 11 and 11 are conducted therebetween in S103.

Thus, the conducting of both the skin-side electrodes 11 and the non-skin-side electrodes 12 indicates that an amount of liquid (urine) sufficient to reach both the spaces between the skin-side electrodes 11 and 11 and between the non-skin-side electrodes 12 and 12 is absorbed by the absorbent body 2. Therefore, in this case, the detection device 50 determines that urination has been performed (S105).

If urination is determined in S105, the detection device 50 issues a warning (alarm) for notifying the user (wearer or caregiver) that urination has been performed (S109). The alarm is performed, for example, by the detection device 50 sounding a buzzer or transmitting alarm information to the information processing apparatus 60 so as to cause a display unit (for example, a display) of the information processing apparatus 60 to display a screen informing that urination has been performed. Alternatively, the alarm information may be transmitted to another terminal through the information processing apparatus 60 and displayed on a display unit of the other terminal. For example, the warning may be output on a display screen of a facility terminal or a terminal device used by a nurse, which is installed in a nurse center and can be linked with a nurse call button. This makes the user easier to appropriately determine the change timing and the like of the absorbent pad 1. However, the alarm (S109) does not necessarily have to be performed.

On the other hand, in S103, if the amount of change in the capacitance value C11 is less than the predetermined magnitude (No in S103), the process proceeds to step S106. The case where the amount of change in the capacitance value C11 is less than the predetermined magnitude means a state in which the pair of skin-side electrodes 11 and 11 are not conducted therebetween. That is, in S102, the pair of non-skin-side electrodes 12 and 12 are conducted therebetween, and, in S103, the pair of skin-side electrodes 11 and 11 are not conducted therebetween.

In this case, the detection device 50 (information processing apparatus 60) determines that a small amount of urine has been excreted (S106). If the amount of urine excreted is small, there is a case where only the non-skin-side electrodes 12 facing the absorbent body 21 come into contact with urine, that is, a case where the non-skin-side electrodes 12 and 12 are conducted. In such a case, it is determined that a small amount of urine has been excreted.

If it is determined in S106 that urination (small amount) has been performed, the detection device 50 issues a warning (alarm) for notifying the user (wearer or a caregiver thereof) that the small amount of urination has been performed (S110). The alarm can be performed in substantially the same manner as in S109, but it is recommended to use a different warning sound or a different screen display, which makes the user easier to recognize the amount of urine excreted.

Next, detection of defecation will be described. Returning to S102 of FIG. 8, if the amount of change in the capacitance value C12 between the non-skin-side electrodes 12 and 12 is less than the predetermined magnitude (No in S102), the detection device 50 determines the amount of change in the capacitance value C11 during the predetermined period based on the detection result of the skin-side capacitance detection process (S104). As a result, if the case where the amount of change in the capacitance value C11 is equal to or larger than the predetermined magnitude (Yes in S104), the process proceeds to step S107. The case where the amount of change in the capacitance value C11 is equal to or larger than the predetermined magnitude means a state in which the pair of skin-side electrodes 11 and 11 are conducted therebetween. That is, it means a state in which the pair of non-skin-side electrodes 12 and 12 are not conducted therebetween in S102 and the pair of skin-side electrodes 11 and 11 are conducted therebetween in S103.

A such phenomenon that only the skin-side electrodes 11 are conducted and the non-skin-side electrodes 12 are not conducted indicates that feces has been excreted in the absorbent pad 1 as illustrated in FIGS. 7A and 7B. Therefore, in this case, the detection device 50 determines that defecation has been performed (S107).

If defecation is determined in S107, the detection device 50 issues a warning (alarm) for notifying the user (wearer or caregiver) that defecation has been performed (S111). The alarm can be performed in substantially the same manner as in S109 and S110. However, it is recommended to use a different warning sound or a different screen display, which makes the user easier to recognize that excrement is feces and that the absorbent pad 1 will be necessary to be changed.

On the other hand, in S104, if the amount of change in the capacitance value C11 is less than the predetermined magnitude (No in S104), the process proceeds to step S108. The case where the amount of change in the capacitance value C11 is less than the predetermined magnitude means a state in which moisture such as urine does not reach between the pair of skin-side electrodes 11 and 11 and does not make conduct between the electrodes. That is, it means a state in which the pair of non-skin-side electrodes 12 and 12 are not conducted therebetween and the pair of skin-side electrodes 11 and 11 are not conducted therebetween.

The reason why both the skin-side electrodes 11 and the non-skin-side electrodes 12 are not conducted is that excretion has not been performed at all or that the amount of excrement is extremely small to an extent that no excrement is detected in each set of electrodes. Therefore, in this case, the detection device 50 determines that excretion has not been performed (S108). It should be noted that, if the amount of excrement is small, there is little influence on the absorption performance of the absorbent pad 1 (absorbent body 2), and therefore, it is less likely to cause a problem even when it is determined that excretion has not been performed. In addition, if it is determined that excretion has not been performed, an alarm or the like is not issued, which does not bother the user.

As described above, in the absorbent pad 1, it is possible to determine with high accuracy whether the excrement is feces or urine, based on the amount of change in the data (capacitance values C11 and C12) detected from the skin-side electrodes 11 and the non-skin-side electrodes 12 within the predetermined period. Further, when urine has been excreted, the excretion can be determined in amount. Accordingly, the user can appropriately determine the change timing of the absorbent pad 1, without taking the trouble of opening and checking the diaper 101 (absorbent article) used by the wearer every time of excretion.

### Second Embodiment

In the second embodiment, a method for determining excrement (determining whether excrement is urine or feces) with higher accuracy, using the absorbent pad 1 described in the first embodiment will be described. It should be noted that the configuration of the apparatus including the absorbent pad 1 (see FIGS. 1 to 5) and the principle of detecting excrement by the electrodes 11 and 12 (see FIGS. 6 to 7) are the same as those of the first embodiment, and therefore description thereof will be omitted.

### Method for Detecting Excrement

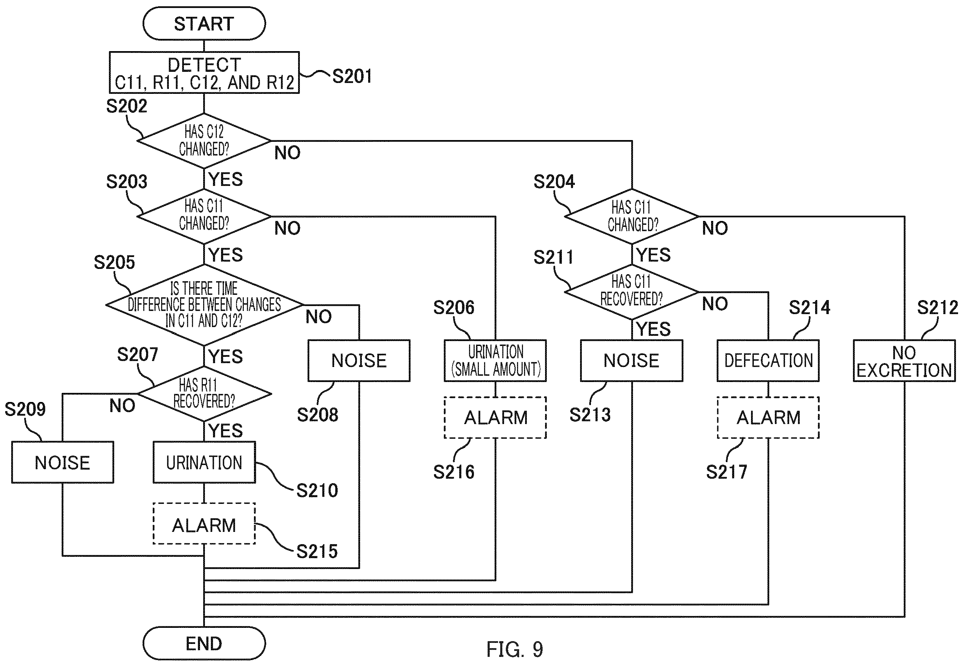

FIG. 9 is a flow chart showing an example of a defecation/urination determination process using the absorbent pad 1 in a second embodiment. When the detection of excrement is started, the detection device 50 intermittently applies a current to each of the electrodes 11 and 12 of the absorbent pad 1. Then, the following detection processes are performed: the non-skin-side capacitance detection process of detecting the magnitude of the capacitance value C12 between the non-skin-side electrodes 12 and 12; and a non-skin-side resistance detection process of detecting the magnitude of the resistance value R12 between the non-skin-side electrodes 12 and 12. Further, the following detection processes are performed: the skin-side capacitance detection process of detecting the magnitude of the capacitance value C11 between the skin-side electrodes 11 and 11; and a skin-side resistance detection process of detecting the magnitude of the resistance value R11 between the skin-side electrodes 11 and 11 (S201).

Next, in the same manner as in S102 to S104 of the first embodiment, the detection device 50 makes a determination for the amount of changes in the capacitance value C12 and the capacitance value C11 detected by each set of the electrodes 11 and 12 during a predetermined period (S202 to S204). The content of the determination is the same as that of the first embodiment.

In the case where S203 in FIG. 9 is determined as Yes, that is, in the case where both the amount of changes in the capacitance value C12 and the capacitance value C11 is equal to or larger than a predetermined value, the detection device 50 determines whether or not a predetermined time difference is generated between the timing when the capacitance value C12 has changed and the timing when the capacitance value C11 has changed (S205). For example, it is determined whether or not there is a slipping of a predetermined time (for example, 0.5 seconds) between the timing when the capacitance value C11 is started to change and the timing when the capacitance value C12 is started to change.

Similar to the case described in S103 of the first embodiment, in the case where S203 is determined as Yes, there is a high possibility that urination is performed on the absorbent pad 1. Here, if urine has been excreted on the skin-side surface of the absorbent body 2, a predetermined time difference should be generated until the urine permeates into the absorbent body 2 from the skin side to the non-skin side. That is, urine excreted in the absorbent pad 1 should come into contact with the non-skin-side electrode 12 on the non-skin side of the absorbent body 2 after a predetermined time has elapsed since the urine comes into contact with the skin-side electrode 11 on the skin side of the absorbent body 2.

Therefore, if there is no predetermined time difference between the timing when the capacitance value C12 has changed and the timing when the capacitance value C11 has changed, that is, if both the capacitance value C12 and the capacitance value C11 have changed at the same timing (No in S205), this behavior is different from the behavior of normal urination. In this case, the detection device 50 determines that the change in the capacitance values C11 and C12 is noise, and determines that urination is not performed (S208).

For example, even if urination is not performed, when the wearer moves the body, there is a possibility that the capacitance values C11 and C12 are changed at the same time due to the body weight of the wearer being temporarily applied to the skin-side electrodes 11 and the non-skin-side electrodes 12 (the wearer's skin being pressed). When the determination of urination is performed based on such a detection result, there is a high possibility that erroneous determination is made. Therefore, if a behavior different from the behavior of normal urination is detected, the detection device 50 does not perform urination determination, and processes the detection result as noise. This makes it possible to enhance the detection accuracy of urination and to suppress erroneous determination.

On the other hand, if a predetermined time difference is generated between the timing when the capacitance value C12 has changed and the timing when the capacitance value C11 has changed (Yes in S205), it is considered that it is the behavior in normal urination, and the process proceeds to step S207.

In S207, the detection device 50 determines whether or not the resistance value R11 detected between the skin-side electrodes 11 and 11 is recovered to the original magnitude within a predetermined time period (S207). For example, the detected value of the resistance value R11 decreases when urination has been performed, and after a predetermined time (for example, 5 minutes) has elapsed, it is determined whether or not the resistance value R11 is recovered to, for example, approximately 20% from the minimum value of the detected value which has decreased when urination has been performed.

When urination has been performed, urine excreted on the skin-side surface of the absorbent body 2 is absorbed by the absorbent body 2 over time. Therefore, immediately after urination, the resistance value R11 decreases due to conducting between the pair of skin-side electrodes 11 and 11. However, when urine is absorbed by the absorbent body 2 after the elapse of a predetermined time, the region between the pair of skin-side electrodes 11 and 11 is brought in a de-conducted state again, and the resistance value R11 is returned to its original magnitude (the resistance value R11 is recovered). Therefore, such a degree of recovery of the resistance value R11 can be utilized as an additional index for determining urination.

In S207, if the resistance value R11 is not recovered after the elapse of the predetermined time (No in S207), this behavior is different from the behavior in normal urination. In this case, the detection device 50 determines that the change in the resistance value R11 is noise and determines that urination is not performed (S209).

Similar to the case described in S208, even if urination is not performed, when the wearer moves the body, there is a possibility that the skin-side electrodes 11 are conducted due to the weight of the wearer being applied to the skin-side electrodes 11 (the wearer's skin is pressed), and the resistance value R11 decreases, and remains and does not recover. When the determination of urination is performed based on such a detection result, there is a high possibility that erroneous determination is made. Therefore, if a behavior different from the behavior of normal urination is detected as described above, the detection device 50 processes the detection result as noise. This makes it possible to enhance the detection accuracy of urination.

On the other hand, if the resistance value R11 is recovered after the elapse of the predetermined time (Yes in S207), it is considered that it is the behavior in normal urination, and the detection device 50 determines that urination has been performed (S210).

If urination is determined in S210, the detection device 50 issues a warning (alarm) for notifying the user (wearer or caregiver) that urination has been performed (S215). The alarm can be performed in a substantially similar manner to that described in S109 of the first embodiment.

Returning to S203 of FIG. 9, if the amount of change in the capacitance value C11 between the skin-side electrodes 11 and 11 is less than the predetermined magnitude (No in S203), the detection device 50 determines that a small amount of urine has been excreted (S206). The reason for this is the same as that described in S106 of the first embodiment.

If it is determined in S206 that urination (small amount) has been performed, the detection device 50 issues a warning (alarm) for notifying the user (wearer or a caregiver thereof) that a small amount of urination has been performed (S216). The alarm can be performed in substantially the same manner as in S215, but it is recommended to use a different warning sound or a different screen display, which makes the user easier to recognize the amount of urine excreted.

Next, detection of defecation will be described. Returning to S204 in FIG. 9, if the amount of change in the capacitance value C11 between the skin-side electrodes 11 and 11 is equal to or larger than the predetermined magnitude (Yes in S204), the detection device 50 determines whether or not the capacitance value C11 is recovered to the original magnitude within the predetermined time period (S211). For example, when defecation has been performed, the detected value of the capacitance value C11 increases, but after the elapse of a predetermined time (for example, 60 seconds), it is determined whether or not the capacitance value C11 is recovered to approximately 120% of the original magnitude.

If defecation has been performed, the feces excreted on the skin-side surface of the absorbent body 2 is less likely to be absorbed by the absorbent body 2, and therefore, the feces continuously remains on the skin-side surface even after the elapse of time. Therefore, after the capacitance value C11 increases due to the passage of electricity between the pair of skin-side electrodes 11 and 11 immediately after defecation, the capacitance value C11 is less likely to change even after a predetermined time has elapsed. That is, if defecation has been performed, the magnitude of the capacitance value C11 is less likely to recover. Therefore, such a degree of recovery of the capacitance value C11 can be utilized as an additional index for determining defecation.

In S211, if the capacitance value C11 has recovered after the elapse of the predetermined time (Yes in S211), this behavior is different from the behavior in normal defecation. In this case, the detection device 50 determines that the change in the capacitance value C11 is noise and determines that defecation is not performed (S213).

On the other hand, if the capacitance value C11 has not recovered even after the elapse of the predetermined time (No in S211), it is considered that it is the behavior in normal defecation, and the detection device 50 determines that defecation has been performed (S214).

Then, if defecation has been determined in S214, the detection device 50 issues a warning (alarm) for notifying the user (wearer or a caregiver thereof) that defecation has been performed (S217).

Further, in S204, the case where the amount of change in the capacitance value C11 between the skin-side electrodes 11 and 11 is less than the predetermined magnitude (No in S204) means a state in which the pair of non-skin-side electrodes 12 and 12 are conducted therebetween and the pair of skin-side electrodes 11 and 11 are conducted therebetween. In this case, the detection device 50 determines that excretion is not performed (S212). As described in S108 of the first embodiment, this is because a phenomenon that neither the skin-side electrodes 11 nor the non-skin-side electrodes 12 are conducted indicates that excretion is not performed at all or that the amount of excrement is extremely small to an extent that no excrement is detected in each set of electrodes.

As described above, in the second embodiment, the timings at which the capacitance values C11 and C12 and the resistance values R11 and R12 are changed and the amount of changes (degree of recovery) after the elapse of the predetermined time are utilized as indexes for determining excretion. Accordingly, the detected data can be removed as noise under predetermined conditions, and excretion can be more accurately determined. Other Embodiments

Although the above embodiments of the present invention have been described, the above-described embodiments are intended to facilitate the understanding of the present invention and are not intended to limit the interpretation of the present invention. Further, it is needless to say that the present invention can be modified or improved without departing from the gist thereof, and the present invention includes an equivalent thereof.

In the above-described embodiment, the non-skin-side electrodes 12 are formed by applying a conductive ink in a strip-shape pattern to the surface of the base sheet 122, but another configuration is also acceptable. For example, the non-skin-side electrodes 12 may be formed by directly applying the conductive ink to the skin-side surface of the leak-proof sheet 4 arranged on the non-skin-side surface of the absorbent body 2. That is, the leak-proof sheet 4 may have a function of the base sheet 122. The leak-proof sheet 4 itself is a liquid-impermeable sheet member, and therefore even in such a configuration, excrement can be detected in the same manner as in a case where the base sheet 122 is provided separately. With the above-described configuration, it is not necessary to separately prepare the base sheet 122, and the manufacturing steps can be simplified. Therefore, the manufacturing costs can be reduced.

### REFERENCE SIGNS LIST

1: absorbent pad (absorbent article),
2: absorbent body,
21: absorbent core, 21A: skin-side core layer, 21B: non-skin-side core layer,
22: core-wrapping sheet,
3: top sheet (liquid-permeable sheet),
4: leak-proof sheet (liquid-impermeable sheet),
5: back sheet (exterior sheet),
10: electrode,
11: skin-side electrode,
111: conductive portion, 112: base sheet (liquid-impermeable sheet member), 113: covering portion,
12: non-skin-side electrode,
121: conductive portion, 122: base sheet (liquid-impermeable sheet member), 123: covering portion,
50: detection device,
51: main body unit, 52: connection unit, 53: data transmitting and receiving unit,
60: information processing apparatus,
101: diaper (disposable diaper, absorbent article)
111: absorbent core, 121: top-surface sheet, 131: back-surface sheet, 141: fastening tape

## Claims

1. An absorbent article (1) having a longitudinal direction, a width direction, and a thickness direction that intersect each other in a stretched state,
the absorbent article (1) comprising:
a liquid-absorbent absorbent body (2);
a liquid-permeable sheet (3) that is arranged on a skin side in the thickness direction with respect to the absorbent body (2);
a liquid-impermeable sheet (4) that is arranged on a non-skin side in the thickness direction with respect to the absorbent body (2);
at least one pair of skin-side electrodes (11) between the liquid-permeable sheet (3) and the absorbent body (2); and
at least one pair of non-skin-side electrodes (12) between the liquid-impermeable sheet (4) and the absorbent body (2); wherein
the at least one pair of non-skin-side electrodes (12) are provided spaced apart from each other in the width direction by a predetermined distance,
the at least one pair of skin-side electrodes (11) are provided spaced apart from each other in the width direction by a distance that is different from the predetermined distance, and
a space between the pair of skin-side electrodes (11) in the width direction is larger than a space between the pair of non-skin-side electrodes (12) in the width direction.

2. The absorbent article (1) according to claim 1, wherein
a space between the pair of skin-side electrodes (11) in the width direction is 40 mm or larger.

3. The absorbent article (1) according to claim 1 or 2, wherein
a space between the pair of non-skin-side electrodes (12) in the width direction is 40 mm or larger.

4. The absorbent article (1) according to any preceding claim, wherein
a presence/absence of excretion is detected based on contact of each of the skin-side electrodes (11) and the non-skin-side electrodes (12) with excrement.

5. The absorbent article (1) according to any preceding claim, wherein
the skin-side electrodes (11) and the non-skin-side electrodes (12) each include:
a conductive portion (111, 121); and
a liquid-impermeable sheet member (112, 122) that is arranged overlaid on the conductive portion (111, 121) in the thickness direction.

6. The absorbent article (1) according to claim 5, wherein
each of the skin-side electrodes (11) and the non-skin-side electrodes (12) include the liquid-impermeable sheet member (112, 122) on an identical side in the thickness direction.

7. The absorbent article (1) according to claim 6, wherein
each of the skin-side electrodes (11) and the non-skin-side electrodes (12) include the liquid-impermeable sheet member (112, 122) on the non-skin side in the thickness direction.

8. The absorbent article (1) according to any one of claims 5 to 7, wherein
a width of the liquid-impermeable sheet member (112, 122) is larger than widths of the skin-side electrode (11) and the non-skin-side electrode (12).

9. The absorbent article (1) according to any one of claims 5 to 8, wherein
a liquid-impermeable region is provided in at least a part of each of the skin-side electrodes (11) and the non-skin-side electrodes (12), on a side in the thickness direction which is opposite to a side where the liquid-impermeable sheet member (112, 122) is provided.

10. The absorbent article according to claim 9, wherein
in the longitudinal direction,
an area of the liquid-impermeable region on a front side with respect to a center is larger than an area of the liquid-impermeable region on a back side with respect to the center.

11. The absorbent article according to any one of claims 1 to 10, wherein
a thickness of the liquid-permeable sheet (3) is thicker than thicknesses of the skin-side electrodes (11) and the non-skin-side electrodes (12).

12. The absorbent article according to any one of claims 1 to 11, wherein
end portions of the skin-side electrodes (11) and the non-skin-side electrodes (12) in the longitudinal direction of the skin-side electrodes (11) and the non-skin-side electrodes (12) are positioned outside at least one of a front end and a back end of the absorbent body (2) in the longitudinal direction.

13. A defecation/urination determination method in an absorbent article (1),
the absorbent article (1) including:
a liquid-absorbent absorbent body (2);
a liquid-permeable sheet (3) that is arranged on a skin side in a thickness direction with respect to the absorbent body (2); and
a liquid-impermeable sheet (4) that is arranged on a non-skin side in the thickness direction with respect to the absorbent body (2), the method comprising:
a skin-side capacitance detection process of detecting a magnitude of a capacitance by a skin-side electrode (11) that is provided between the liquid-permeable sheet (3) and the absorbent body (2);
a non-skin-side capacitance detection process of detecting a magnitude of a capacitance by a non-skin-side electrode (12) that is provided between the liquid-impermeable sheet (4) and the absorbent body (2); and
a determination process of determining whether excrement that has been excreted on the absorbent article (1) is feces or urine based on
an amount of change in the capacitance that has been detected in the skin-side capacitance detection process during a predetermined period and
an amount of change in the capacitance that has been detected in the non-skin-side capacitance detection process during the predetermined period
wherein
at least one pair of non-skin-side electrodes (12) are provided spaced apart from each other in the width direction by a predetermined distance,
at least one pair of skin-side electrodes (11) are provided spaced apart from each other in the width direction by a distance that is different from the predetermined distance, and
a space between the pair of skin-side electrodes (11) in the width direction is larger than a space between the pair of non-skin-side electrodes (12) in the width direction.

14. The defecation/urination determination method according to claim 13, wherein
if the amount of change in the capacitance that has been detected by the skin-side electrode (11) during the predetermined period is equal to or larger than a predetermined value, and
if the amount of change in the capacitance that has been detected by the non-skin-side electrode (12) during the predetermined period is equal to or larger than the predetermined value,
it is determined that urine has been excreted, and
if the amount of change in the capacitance that has been detected by the skin-side electrode (11) during the predetermined period is equal to or larger than the predetermined value, and
if the amount of change in the capacitance that has been detected by the non-skin-side electrode (12) during the predetermined period is less than the predetermined value,
it is determined that feces has been excreted.

15. The defecation/urination determination method according to claim 14, wherein
concerning a timing at which the capacitance that has been detected by the skin-side electrode (11) has changed during the predetermined period, and
concerning a timing at which the capacitance that has been detected by the non-skin-side electrode (12) has changed during the predetermined period,
a difference between these timings is utilized as an additional index for determining urination.

16. The defecation/urination determination method according to claim 15, wherein
concerning the timing at which the capacitance that has been detected by the skin-side electrode (11) has changed during the predetermined period, and
concerning the timing at which the capacitance that has been detected by the non-skin-side electrode (12) has changed during the predetermined period,
in a case where these timings are simultaneous, this case is determined as noise.

17. The defecation/urination determination method according to any one of claims 14 to 16, wherein
the method further comprises a skin-side resistance detection process of detecting a magnitude of a resistance value by the skin-side electrode (11), and
concerning a degree of recovery during the predetermined period after the resistance value that has been detected by the skin-side electrode (11) has changed,
the degree is utilized as an additional index for determining urination.

18. The defecation/urination determination method according to any one of claims 14 to 17, wherein
concerning a degree of recovery during the predetermined period after the capacitance value that has been detected by the skin-side electrode (11) has changed,
the degree is utilized as an additional index for determining defecation.

## Patentansprüche

1. Absorbierender Artikel (1), der eine Längsrichtung, eine Breitenrichtung und eine Dickenrichtung aufweist, die in einem gedehnten Zustand einander schneiden,
wobei der absorbierende Artikel (1) Folgendes umfasst:
einen flüssigkeitsabsorbierenden Saugkörper (2);
eine flüssigkeitsdurchlässige Lage (3), die in der Dickenrichtung in Bezug auf den Saugkörper (2) auf einer Hautseite angeordnet ist;
eine flüssigkeitsundurchlässige Lage (4), die in der Dickenrichtung in Bezug auf den Saugkörper (2) auf einer Nicht-Hautseite angeordnet ist;
mindestens ein Paar von Hautseitenelektroden (11) zwischen der flüssigkeitsdurchlässigen Lage (3) und dem Saugkörper (2); und
mindestens ein Paar von Nicht-Hautseitenelektroden (12) zwischen der flüssigkeitsundurchlässigen Lage (4) und dem Saugkörper (2);
wobei
das mindestens eine Paar von Nicht-Hautseitenelektroden (12) durch einen vorbestimmten Abstand in der Breitenrichtung beabstandet voneinander bereitgestellt ist,
das mindestens eine Paar von Hautseitenelektroden (11) durch einen Abstand, der sich von dem vorbestimmten Abstand unterscheidet, in der Breitenrichtung beabstandet voneinander bereitgestellt ist,
und
ein Zwischenraum zwischen dem Paar von Hautseitenelektroden (11) in der Breitenrichtung größer ist als ein Zwischenraum zwischen dem Paar von Nicht-Hautseitenelektroden (12) in der Breitenrichtung.

2. Absorbierender Artikel (1) nach Anspruch 1, wobei
ein Zwischenraum zwischen dem Paar von Hautseitenelektroden (11) in der Breitenrichtung 40 mm oder mehr beträgt.

3. Absorbierender Artikel (1) nach Anspruch 1 oder 2, wobei
ein Zwischenraum zwischen dem Paar von Nicht-Hautseitenelektroden (12) in der Breitenrichtung 40 mm oder mehr beträgt.

4. Absorbierender Artikel (1) nach einem vorstehenden Anspruch, wobei
ein Vorhandensein/Nichtvorhandensein von Ausscheidung basierend auf Kontakt von jeder der Hautseitenelektroden (11) und der Nicht-Hautseitenelektroden (12) mit Ausscheidung detektiert wird.

5. Absorbierender Artikel (1) nach einem vorstehenden Anspruch, wobei
die Hautseitenelektroden (11) und die Nicht-Hautseitenelektroden (12) jeweils Folgendes einschließen:
einen leitfähigen Teil (111, 121); und
ein flüssigkeitsundurchlässiges Lagenelement (112, 122), das überlagernd mit dem leitfähigen Teil (111, 121) in der Dickenrichtung angeordnet ist.

6. Absorbierender Artikel (1) nach Anspruch 5, wobei
jede der Hautseitenelektroden (11) und der Nicht-Hautseitenelektroden (12) das flüssigkeitsundurchlässige Lagenelement (112, 122) auf einer gleichen Seite in der Dickenrichtung einschließt.

7. Absorbierender Artikel (1) nach Anspruch 6, wobei
jede der Hautseitenelektroden (11) und der Nicht-Hautseitenelektroden (12) das flüssigkeitsundurchlässige Lagenelement (112, 122) auf der Nicht-Hautseite in der Dickenrichtung einschließt.

8. Absorbierender Artikel (1) nach einem der Ansprüche 5 bis 7, wobei
eine Breite des flüssigkeitsundurchlässigen Lagenelements (112, 122) größer ist als die Breiten der Hautseitenelektrode (11) und der Nicht-Hautseitenelektrode (12).

9. Absorbierender Artikel (1) nach einem der Ansprüche 5 bis 8, wobei
ein flüssigkeitsundurchlässiger Bereich in zumindest einem Teil von jeder der Hautseitenelektroden (11) und der Nicht-Hautseitenelektroden (12) auf einer Seite in der Dickenrichtung bereitgestellt ist, die einer Seite gegenüberliegt, wo das flüssigkeitsundurchlässige Lagenelement (112, 122) bereitgestellt ist.

10. Absorbierender Artikel nach Anspruch 9, wobei
in der Längsrichtung
eine Fläche des flüssigkeitsundurchlässigen Bereichs auf einer Vorderseite in Bezug auf eine Mitte größer ist als eine Fläche des flüssigkeitsundurchlässigen Bereichs auf einer Rückenseite in Bezug auf die Mitte.

11. Absorbierender Artikel nach einem der Ansprüche 1 bis 10, wobei
eine Dicke der flüssigkeitsdurchlässigen Lage (3) dicker ist als die Dicken der Hautseitenelektroden (11) und der Nicht-Hautseitenelektroden (12).

12. Absorbierender Artikel nach einem der Ansprüche 1 bis 11, wobei
Endteile der Hautseitenelektroden (11) und der Nicht-Hautseitenelektroden (12) in der Längsrichtung der Hautseitenelektroden (11) und der Nicht-Hautseitenelektroden (12) außerhalb von mindestens einem eines Vorderendes und eines Rückenendes des Saugkörpers (2) in der Längsrichtung positioniert sind.

13. Verfahren zur Bestimmung von Stuhlgang/Urinieren in einem absorbierenden Artikel (1),
wobei der absorbierende Artikel (1) Folgendes einschließt:
einen flüssigkeitsabsorbierenden Saugkörper (2);
eine flüssigkeitsdurchlässige Lage (3), die in einer Dickenrichtung in Bezug auf den Saugkörper (2) auf einer Hautseite angeordnet ist; und
eine flüssigkeitsundurchlässige Lage (4), die in der Dickenrichtung in Bezug auf den Saugkörper (2) auf einer Nicht-Hautseite angeordnet ist, wobei das Verfahren Folgendes umfasst:
einen Kapazitätsdetektionsvorgang auf der Hautseite des Detektierens einer Größenordnung einer Kapazität durch eine Hautseitenelektrode (11), die zwischen der flüssigkeitsdurchlässigen Lage (3) und dem Saugkörper (2) bereitgestellt ist;
einen Kapazitätsdetektionsvorgang auf der Nicht-Hautseite des Detektierens einer Größenordnung einer Kapazität durch eine Nicht-Hautseitenelektrode (12), die zwischen der flüssigkeitsundurchlässigen Lage (4) und dem Saugkörper (2) bereitgestellt ist; und
einen Bestimmungsvorgang des Bestimmens, ob Ausscheidung, die auf den absorbierenden Artikel (1) ausgeschieden wurde, Kot oder Urin ist, basierend auf
einem Ausmaß der Änderung der Kapazität, das in dem Kapazitätsdetektionsvorgang auf der Hautseite während einer vorbestimmten Zeitdauer detektiert wurde, und
einem Ausmaß der Änderung der Kapazität, das in dem Kapazitätsdetektionsvorgang auf der Nicht-Hautseite während der vorbestimmten Zeitdauer detektiert wurde,
wobei
mindestens ein Paar von Nicht-Hautseitenelektroden (12) durch einen vorbestimmten Abstand in der Breitenrichtung beabstandet voneinander bereitgestellt ist,
mindestens ein Paar von Hautseitenelektroden (11) durch einen Abstand, der sich von dem vorbestimmten Abstand unterscheidet, in der Breitenrichtung beabstandet voneinander bereitgestellt ist, und
ein Zwischenraum zwischen dem Paar von Hautseitenelektroden (11) in der Breitenrichtung größer ist als ein Zwischenraum zwischen dem Paar von Nicht-Hautseitenelektroden (12) in der Breitenrichtung.

14. Verfahren zur Bestimmung von Stuhlgang/Urinieren nach Anspruch 13, wobei,
wenn das Ausmaß der Änderung der Kapazität, das durch die Hautseitenelektrode (11) während der vorbestimmten Zeitdauer detektiert wurde, gleich oder größer ist als ein vorbestimmter Wert und
wenn das Ausmaß der Änderung der Kapazität, das durch die Nicht-Hautseitenelektrode (12) während der vorbestimmten Zeitdauer detektiert wurde, gleich oder größer ist als der vorbestimmte Wert,
bestimmt wird, dass Urin ausgeschieden wurde, und,
wenn das Ausmaß der Änderung der Kapazität, das durch die Hautseitenelektrode (11) während der vorbestimmten Zeitdauer detektiert wurde, gleich oder größer ist als der vorbestimmte Wert und
wenn das Ausmaß der Änderung der Kapazität, das durch die Nicht-Hautseitenelektrode (12) während der vorbestimmten Zeitdauer detektiert wurde, kleiner ist als der vorbestimmte Wert,
bestimmt wird, dass Kot ausgeschieden wurde.

15. Verfahren zur Bestimmung von Stuhlgang/Urinieren nach Anspruch 14, wobei,
hinsichtlich eines Zeitpunkts, an dem sich die Kapazität, die durch die Hautseitenelektrode (11) detektiert wurde, während der vorbestimmten Zeitdauer geändert hat, und
hinsichtlich eines Zeitpunkts, an dem sich die Kapazität, die durch die Nicht-Hautseitenelektrode (12) detektiert wurde, während der vorbestimmten Zeitdauer geändert hat,
ein Unterschied zwischen diesen Zeitpunkten als ein zusätzliches Anzeichen für die Bestimmung von Urinieren genutzt wird.

16. Verfahren zur Bestimmung von Stuhlgang/Urinieren nach Anspruch 15, wobei,
hinsichtlich des Zeitpunkts, an dem sich die Kapazität, die durch die Hautseitenelektrode (11) detektiert wurde, während der vorbestimmten Zeitdauer geändert hat, und
hinsichtlich des Zeitpunkts, an dem sich die Kapazität, die durch die Nicht-Hautseitenelektrode (12) detektiert wurde, während der vorbestimmten Zeitdauer geändert hat,
in einem Fall, wo diese Zeitpunkte zeitgleich sind, dieser Fall als Rauschen bestimmt wird.

17. Verfahren zur Bestimmung von Stuhlgang/Urinieren nach einem der Ansprüche 14 bis 16, wobei
das Verfahren weiter einen Widerstandsdetektionsvorgang auf der Hautseite des Detektierens einer Größenordnung eines Widerstandswerts durch die Hautseitenelektrode (11) umfasst und,
hinsichtlich eines Grads der Wiederherstellung während der vorbestimmten Zeitdauer, nachdem sich der Widerstandswert, der durch die Hautseitenelektrode (11) detektiert wurde, verändert hat,
der Grad als ein zusätzliches Anzeichen für die Bestimmung von Urinieren genutzt wird.

18. Verfahren zur Bestimmung von Stuhlgang/Urinieren nach einem der Ansprüche 14 bis 17, wobei,
hinsichtlich eines Grads der Wiederherstellung während der vorbestimmten Zeitdauer, nachdem sich der Kapazitätswert, der durch die Hautseitenelektrode (11) detektiert wurde, verändert hat,
der Grad als ein zusätzliches Anzeichen für die Bestimmung von Stuhlgang genutzt wird.

## Revendications

1. Article absorbant (1) ayant une direction longitudinale, une direction allant dans le sens de la largeur et une direction allant dans le sens de l'épaisseur qui se croisent les unes les autres dans un état étiré,
l'article absorbant (1), comportant :
un corps absorbant qui absorbe les liquides (2) ;
une feuille perméable aux liquides (3) qui est agencée sur un côté orienté vers la peau dans la direction allant dans le sens de l'épaisseur par rapport au corps absorbant (2) ;
une feuille imperméable aux liquides (4) qui est agencée sur un côté non orienté vers la peau dans la direction allant dans le sens de l'épaisseur par rapport au corps absorbant (2) ;
au moins une paire d'électrodes côté orienté vers la peau (11) entre la feuille perméable aux liquides (3) et le corps absorbant (2) ; et
au moins une paire d'électrodes côté non orienté vers la peau (12) entre la feuille imperméable aux liquides (4) et le corps absorbant (2) ;
dans lequel
les électrodes de ladite au moins une paire d'électrodes côté non orienté vers la peau (12) sont mises en œuvre de manière espacée l'une par rapport à l'autre dans la direction allant dans le sens de la largeur selon une distance prédéterminée,
les électrodes de ladite au moins une paires d'électrodes côté orienté vers la peau (11) sont mises en œuvre de manière espacée l'une par rapport à l'autre dans la direction allant dans le sens de la largeur selon une distance qui est différente de la distance prédéterminée, et
un espace entre les électrodes de la paire d'électrodes côté orienté vers la peau (11) dans la direction allant dans le sens de la largeur est supérieur par rapport à un espace entre les électrodes de la paire d'électrodes côté non orienté vers la peau (12) dans la direction allant dans le sens de la largeur.

2. Article absorbant (1) selon la revendication 1, dans lequel
un espace entre les électrodes de la paire d'électrodes côté orienté vers la peau (11) dans la direction allant dans le sens de la largeur est supérieur ou égal à 40 mm.

3. Article absorbant (1) selon la revendication 1 ou la revendication 2, dans lequel
un espace entre les électrodes de la paire d'électrodes côté non orienté vers la peau (12) dans la direction allant dans le sens de la largeur est supérieur ou égal à 40 mm.

4. Article absorbant (1) selon l'une quelconque des revendications précédentes, dans lequel
une présence/absence d'excrétion est détectée sur la base d'un contact de chacune des électrodes côté orienté vers la peau (11) et des électrodes côté non orienté vers la peau (12) avec les excréments.

5. Article absorbant (1) selon l'une quelconque des revendications précédentes, dans lequel
les électrodes côté orienté vers la peau (11) et les électrodes côté non orienté vers la peau (12) comprennent chacune :
une partie conductrice (111, 121) ; et
un élément formant feuille imperméable aux liquides (112, 122) qui est agencé de manière à recouvrir la partie conductrice (111, 121) dans la direction allant dans le sens de l'épaisseur.

6. Article absorbant (1) selon la revendication 5, dans lequel
chacune des électrodes côté orienté vers la peau (11) et des électrodes côté non orienté vers la peau (12) comprend l'élément formant feuille imperméable aux liquides (112, 122) sur un côté identique dans la direction allant dans le sens de l'épaisseur.

7. Article absorbant (1) selon la revendication 6, dans lequel
chacune des électrodes côté orienté vers la peau (11) et des électrodes côté non orienté vers la peau (12) comprend l'élément formant feuille imperméable aux liquides (112, 122) sur le côté non orienté vers la peau dans la direction allant dans le sens de l'épaisseur.

8. Article absorbant (1) selon l'une quelconque des revendications 5 à 7, dans lequel
une largeur de l'élément formant feuille imperméable aux liquides (112, 122) est supérieure aux largeurs de l'électrode côté orienté vers la peau (11) et de l'électrode côté non orienté vers la peau (12).

9. Article absorbant (1) selon l'une quelconque des revendications 5 à 8, dans lequel
une région imperméable aux liquides est mise en œuvre dans au moins une partie de chacune des électrodes côté orienté vers la peau (11) et des électrodes côté non orienté vers la peau (12), sur un côté dans la direction allant dans le sens de l'épaisseur qui est opposé à un côté où l'élément formant feuille imperméable aux liquides (112, 122) est mis en œuvre.

10. Article absorbant selon la revendication 9, dans lequel
dans la direction longitudinale,
une superficie de la région imperméable aux liquides sur un côté avant par rapport à un centre est supérieure à une superficie de la région imperméable aux liquides sur un côté arrière par rapport au centre.

11. Article absorbant selon l'une quelconque des revendications 1 à 10, dans lequel
une épaisseur de la feuille perméable aux liquides (3) est plus épaisse que les épaisseurs des électrodes côté orienté vers la peau (11) et des électrodes côté non orienté vers la peau (12).

12. Article absorbant selon l'une quelconque des revendications 1 à 11, dans lequel
des parties d'extrémité des électrodes côté orienté vers la peau (11) et des électrodes côté non orienté vers la peau (12) dans la direction longitudinale des électrodes côté orienté vers la peau (11) et des électrodes côté non orienté vers la peau (12) sont positionnées à l'extérieur d'au moins l'une parmi une extrémité avant et une extrémité arrière du corps absorbant (2) dans la direction longitudinale.

13. Procédé de détermination de défécation/de miction dans un article absorbant (1),
l'article absorbant (1) comprenant :
un corps absorbant qui absorbe les liquides (2) ;
une feuille perméable aux liquides (3) qui est agencée sur un côté orienté vers la peau par rapport au corps absorbant (2) ; et
une feuille imperméable aux liquides (4) qui est agencée sur un côté non orienté vers la peau dans la direction allant dans le sens de l'épaisseur par rapport au corps absorbant (2), le procédé comportant :
un processus de détection de capacité côté orienté vers la peau permettant de détecter une amplitude d'une capacité par une électrode côté orienté vers la peau (11) qui est mise en œuvre entre la feuille perméable aux liquides (3) et le corps absorbant (2) ;
un processus de détection de capacité côté non orienté vers la peau permettant de détecter une amplitude d'une capacité par une électrode côté non orienté vers la peau (12) qui est mise en œuvre entre la feuille imperméable aux liquides (4) et le corps absorbant (2) ; et
un processus de détermination permettant de déterminer si les excréments qui ont été excrétés sur l'article absorbant (1) sont des matières fécales ou de l'urine en se basant sur
une quantité de changement au niveau de la capacité qui a été détectée au cours du processus de détection de capacité côté orienté vers la peau pendant une période prédéterminée et
une quantité de changement au niveau de la capacité qui a été détectée au cours du processus de détection de capacité côté non orienté vers la peau au cours de la période prédéterminée
dans lequel
les électrodes d'au moins une paire d'électrodes côté non orienté vers la peau (12) sont mises en œuvre de manière espacée l'une par rapport à l'autre dans la direction allant dans le sens de la largeur selon une distance prédéterminée,
les électrodes d'au moins une paire d'électrodes côté orienté vers la peau (11) sont mises en œuvre de manière espacée l'une par rapport à l'autre dans la direction allant dans le sens de la largeur selon une distance qui est différente de la distance prédéterminée, et
un espace entre la paire d'électrodes côté orienté vers la peau (11) dans la direction allant dans le sens de la largeur est supérieur par rapport à un espace entre la paire d'électrodes côté non orienté vers la peau (12) dans la direction allant dans le sens de la largeur.

14. Procédé de détermination de défécation/de miction selon la revendication 13, dans lequel
si la quantité de changement au niveau de la capacité qui a été détectée par l'électrode côté orienté vers la peau (11) au cours de la période prédéterminée est égale ou supérieure à une valeur prédéterminée, et
si la quantité de changement au niveau de la capacité qui a été détectée par l'électrode côté non orienté vers la peau (12) au cours de la période prédéterminée est égale ou supérieure à la valeur prédéterminée,
il est déterminé que de l'urine a été excrétée, et
si la quantité de changement au niveau de la capacité qui a été détectée par l'électrode côté orienté vers la peau (11) au cours de la période prédéterminée est égale ou supérieure à la valeur prédéterminée, et
si la quantité de changement au niveau de la capacité qui a été détectée par l'électrode côté non orienté vers la peau (12) au cours de la période prédéterminée est inférieure à la valeur prédéterminée,
il est déterminé que des matières fécales ont été excrétées.

15. Procédé de détermination de défécation/de miction selon la revendication 14, dans lequel
en ce qui concerne un moment auquel la capacité qui a été détectée par l'électrode côté orienté vers la peau (11) a changé au cours de la période prédéterminée, et
en ce qui concerne un moment auquel la capacité qui a été détectée par l'électrode côté non orienté vers la peau (12) a changé au cours de la période prédéterminée,
une différence entre ces moments est utilisée comme indice supplémentaire servant à déterminer la miction.

16. Procédé de détermination de défécation/de miction selon la revendication 15, dans lequel
en ce qui concerne le moment auquel la capacité qui a été détectée par l'électrode côté orienté vers la peau (11) a changé au cours de la période prédéterminée, et
en ce qui concerne le moment auquel la capacité qui a été détectée par l'électrode côté non orienté vers la peau (12) a changé au cours de la période prédéterminée,
dans un cas de figure où ces moments sont simultanées, ce cas de figure est déterminé comme étant du bruit.

17. Procédé de détermination de défécation/de miction selon l'une quelconque des revendications 14 à 16, dans lequel
le procédé comporte par ailleurs un processus de détection de résistance côté orienté vers la peau pour détecter une amplitude d'une valeur de résistance par l'électrode côté orienté vers la peau (11), et
en ce qui concerne un degré de récupération au cours de la période prédéterminée après que la valeur de résistance qui a été détectée par l'électrode côté orienté vers la peau (11) a changé,
le degré est utilisé comme indice supplémentaire pour déterminer la miction.

18. Procédé de détermination de défécation/de miction selon l'une quelconque des revendications 14 à 17, dans lequel
en ce qui concerne un degré de récupération au cours de la période prédéterminée après que la valeur de capacité qui a été détectée par l'électrode côté orienté vers la peau (11) a changé,
le degré est utilisé comme indice supplémentaire pour déterminer la défécation.
